(19) Europäisches Patentamt
European Patent Office
Office européen des brevets

(11) EP 1 325 001 B1

(12) EUROPÄISCHE PATENTSCHRIFT

(45) Veröffentlichungstag und Bekanntmachung des Hinweises auf die Patenterteilung:
**18.02.2004 Patentblatt 2004/08**

(21) Anmeldenummer: **01982374.9**

(22) Anmeldetag: **28.09.2001**

(51) Int Cl.[7]: **C07D 451/10**, A61K 31/46, A61P 43/00

(86) Internationale Anmeldenummer:
**PCT/EP2001/011226**

(87) Internationale Veröffentlichungsnummer:
**WO 2002/032899 (25.04.2002 Gazette 2002/17)**

(54) **ALS ARZNEIMITTEL EINSETZBARE ANTICHOLINERGIKA SOWIE VERFAHREN ZU DEREN HERSTELLUNG**

ANTICHOLINERGIC AGENTS THAT CAN BE USED AS MEDICAMENTS AND METHOD FOR THE PRODUCTION THEREOF

AGENTS ANTICHOLINERGIQUES POUVANT ETRE EMPLOYES EN TANT QUE MEDICAMENTS, ET PROCEDES DE FABRICATION

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **14.10.2000 DE 10050994**

(43) Veröffentlichungstag der Anmeldung:
**09.07.2003 Patentblatt 2003/28**

(60) Teilanmeldung:
**03023933.9 / 1 382 606**

(73) Patentinhaber: **BOEHRINGER INGELHEIM PHARMA GMBH & CO. KG
55216 Ingelheim am Rhein (DE)**

(72) Erfinder:
- **MEISSNER, Helmut
55218 Ingelheim (DE)**
- **MORSCHHÄUSER, Gerd
88400 Biberach (DE)**
- **PIEPER, Michael, Paul
88400 Biberach (DE)**
- **POHL, Gerald
88400 Biberach (DE)**
- **REICHL, Richard
55435 Gau-Algesheim (DE)**
- **SPECK, Georg
55218 Ingelheim am Rhein (DE)**
- **BANHOLZER, Rolf
70597 Stuttgart (DE)**

(56) Entgegenhaltungen:
**WO-A-92/16528 DE-A- 4 003 270**

- **CHEMICAL ABSTRACTS, vol. 122, no. 17, 24. April 1995 (1995-04-24) Columbus, Ohio, US; abstract no. 204554f, SCAPECCHI, S. ET AL.: "Dialkylaminoalkyl esters of 2,2,-diphenyl-2-alkylthioacetic acids: a new class of potent and functionally selective muscarinic antagonists" XP002192832 -& DATABASE CHEMICAL ABSTRACTS [Online] CA 122:204554, XP002192841 & BIOORG. MED. CHEM., Bd. 2, Nr. 10, - 1994 Seiten 1061-1074,**
- **CHEMICAL ABSTRACTS, vol. 82, no. 9, 3. März 1975 (1975-03-03) Columbus, Ohio, US; abstract no. 51342p, ABRAMSON, F.B. ET AL.: "Relations between chemical structure and affinity for postganglionic acetylcholine receptors of the guinea-pig ileum" XP002192833 -& DATABASE CHEMICAL ABSTRACTS [Online] CA 82:51342, XP002192842 & BR. J. PHARMACOL., Bd. 51, Nr. 1, - 1974 Seiten 81-93,**
- **CHEMICAL ABSTRACTS, vol. 77, no. 21, 20. November 1972 (1972-11-20) Columbus, Ohio, US; abstract no. 135027z, KORETSKAYA, N.I. ET AL.: "Synthesis and pharmacological study of tropine esters of alpha-substituted tropic acid" XP002192834 -& DATABASE CHEMICAL ABSTRACTS [Online] CA 77:135027, XP002192843 & KHIM.-FARM. ZH., Bd. 6, Nr. 7, - 1972 Seiten 3-8,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- **CHEMICAL ABSTRACTS, vol. 128, no. 21, 25. Mai 1998 (1998-05-25) Columbus, Ohio, US; abstract no. 252519u, XU, RONG ET AL.: "Synthesis, antimuscarinic activity and quantitative structure-activity relationship (QSAR) of tropinyl and ppiperidinyl esters" XP002192835 & CHEM. PHARM. BULL., Bd. 46, Nr. 2, - 1998 Seiten 231-241, -& DATABASE CHEMICAL ABSTRACTS [Online] CA 128:252519 , XP002192844**
- **CHEMICAL ABSTRACTS, vol. 126, no. 22, 2. Juni 1997 (1997-06-02) Columbus, Ohio, US; abstract no. 292958b, XU, RONG ET AL.: "Structure-hydrolyzability relationships in a series of piperidinyl and tropinyl esters with antimuscarinic activity" XP002192836 & CHEM. PHARM. BULL., Bd. 45, Nr. 3, - 1997 Seiten 476-481, -& DATABASE CHEMICAL ABSTRACTS [Online] CA 126:292958, XP002192845**
- **CHEMICAL ABSTRACTS, vol. 90, no. 23, 4. Juni 1979 (1979-06-04) Columbus, Ohio, US; abstract no. 179952v, MASHKOVSKII, M.D. ET AL.: "Antihistaminic and antiserotonin properties of new tropine esters" XP002192837 -& DATABASE CHEMICAL ABSTRACTS [Online] CA 90:179952, XP002192846 & FARMAKOL. TOKSIKOL. (MOSCOW), Bd. 42, Nr. 1, - 1979 Seiten 3-7,**
- **CHEMICAL ABSTRACTS, vol. 85, no. 13, 27. September 1976 (1976-09-27) Columbus, Ohio, US; abstract no. 87108t, FEHER, O. ET AL.: "The effect of tertiary tropine derivatives on cerebral convulsions" XP002192838 & ACTA BIOL.(SZEGED), Bd. 21, Nr. 1-4, - 1975 Seiten 113-125, -& DATABASE CHEMICAL ABSTRACTS [Online] CA 85:87108, XP002192847**
- **CHEMICAL ABSTRACTS, vol. 77, no. 5, 31. Juli 1972 (1972-07-31) Columbus, Ohio, US; abstract no. 28759t, MASHKOVSKII, M.D. ET AL.: "Cholinolytic activity of hydroxymethyl analogs of atropine and tropacine" XP002192839 & FARMAKOL. TOKSIKOL. (MOSCOW), Bd. 35, Nr. 2, - 1972 Seiten 155-159, -& DATABASE CHEMICAL ABSTRACTS [Online] CA 77:28759t, XP002192848**
- **CHEMICAL ABSTRACTS, vol. 67, no. 15, 9. Oktober 1967 (1967-10-09) Columbus, Ohio, US; abstract no. 73724g, N. A. ZAKHAROVA ET AL.: "Esters of trine, 1-diethylamino)-2-propanol, and beta-(diethylamino)ethanol" XP002192840 & ZH. ORG. KHIM., Bd. 3, Nr. 6, - 1967 Seiten 1128-1136, -& DATABASE CHEMICAL ABSTRACTS [Online] CA 67:73724, XP002192849**

## Beschreibung

Die vorliegende Erfindung betrifft neue Anticholinergika der allgemeinen Formel 1

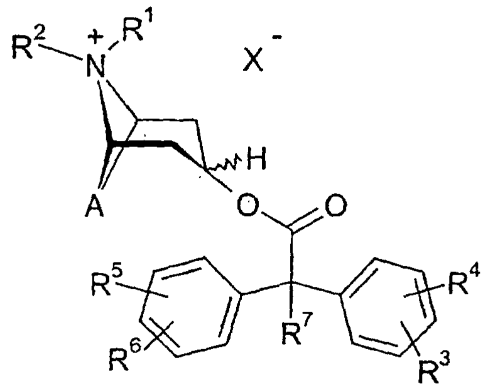

1

worin A, $X^-$ und die Reste $R^1$, $R^2$, $R^3$, $R^4$, $R^5$ ,$R^6$ und $R^7$, die in den Ansprüchen und in der Beschreibung genannten Bedeutungen haben können, Verfahren zu deren Herstellung sowie deren Verwendung als Arzneimittel.

Hintergrund der Erfindung

Anticholinergika können bei einer Vielzahl von Erkrankungen therapeutisch sinnvoll eingesetzt werden. Hervorzuheben sind hier beispielsweise die Therapie von Asthma oder COPD (chronic obstructive pulmonary disease = chronisch obstruktive Lungenerkrankung). Zur Therapie dieser Erkrankungen werden durch die WO 92/16528 Anticholinergika vorgeschlagen, die ein Scopin-, Tropenol- oder auch Tropin-Grundgerüst aufweisen.

Die der WO 92/16528 zugrunde liegende Aufgabe zielt auf die Bereitstellung von anticholinerg wirksamen Verbindungen, die durch eine lang andauernde Wirksamkeit gekennzeichnet sind. Zur Lösung dieser Aufgabe werden durch die WO 92/16528 unter anderem Benzilsäureester des Scopins, Tropenols oder auch Tropins offenbart.

Zur Therapie chronischer Erkrankungen ist es häufig wünschenswert, Arzneimittel mit einer längeren Wirkungsdauer bereitzustellen. Hierdurch kann in der Regel gewährleistet werden, daß die zur Erzielung des therapeutischen Effekts erforderliche Konzentration des Wirkstoffs im Organismus über einen längeren Zeitraum gegeben ist, ohne eine allzu häufige, wiederholte Gabe des Arzneimittels durchführen zu müssen. Die Applikation eines Wirkstoffs in längeren zeitlichen Abständen trägt im übrigen in hohem Maße zum Wohlbefinden des Patienten bei. Besonders wünschenswert ist die Bereitstellung eines Arzneimittels, welches therapeutisch sinnvoll durch einmalige Applikation pro Tag (Einmalgabe) eingesetzt werden kann. Eine einmal pro Tag erfolgende Anwendung hat den Vorteil, daß der Patient sich relativ schnell an die regelmäßige Einnahme des Medikaments zu bestimmten Tageszeiten gewöhnen kann.

Um als einmal täglich anwendbares Medikament zum Einsatz kommen zu können, sind an den zu applizierenden Wirkstoff besondere Anforderungen zu stellen. Zunächst sollte der nach Gabe des Arzneimittels erwünschte Wirkungseintritt relativ schnell erfolgen und im Idealfall über einen sich daran anschließenden längeren Zeitraum eine möglichst konstante Wirksamkeit aufweisen. Andererseits sollte die Wirkdauer des Arzneimittels einen Zeitraum von etwa einem Tag nicht wesentlich überschreiten. Im Idealfall zeigt ein Wirkstoff ein derart geartetes Wirkungsprofil, daß sich die Herstellung eines einmal täglich applizierbaren Arzneimittels, welches den Wirkstoff in therapeutisch sinnvollen Dosen enthält, gezielt steuern läßt.

Es wurde gefunden, daß die in der WO 92/16528 offenbarten Benzilsäureester des Scopins, Tropenols oder auch Tropins diesen erhöhten Anforderungen nicht genügen. Sie sind aufgrund ihrer extrem langen Wirkungsdauer, die den vorstehend genannten Zeitraum von etwa einem.Tag deutlich überschreiten, nicht als Einmalgabe pro Tag therapeutisch nutzbar.

Es ist daher Aufgabe der vorliegenden Erfindung, neue Anticholinergika bereitzustellen, die aufgrund ihres Wirkungsprofils die Herstellung eines einmal täglich applizierbaren Arzneimittels erlauben. Es ist femer Aufgabe der Erfindung, Verbindungen bereitzustellen, die durch einen relativ raschen Wirkungseintritt gekennzeichnet sind. Es ist des weiteren Aufgabe der Erfindung, Verbindungen bereitzustellen, die nach raschem Wirkungseintritt über einen sich daran anschließenden längeren Zeitraum eine möglichst konstante Wirksamkeit aufweisen. Ferner ist es Aufgabe der Erfindung, Verbindungen bereitzustellen, deren Wirkdauer einen Zeitraum von etwa einem Tag bei therapeutisch sinnvoll einsetzbaren Dosierungen nicht wesentlich überschreitet. Schließlich ist es Aufgabe der Erfindung, Verbindungen

bereitzustellen, die ein Wirkungsprofil aufweisen, welches eine gute Steuerbarkeit des therapeutischen Effekts (das heißt volle therapeutische Wirkung ohne Nebenwirkung durch Kumulation der Substanz im Organismus) gewährleistet.

Detaillierte Beschreibung der Erfindung

Überraschenderweise wurde gefunden, daß die vorstehend genannten Aufgaben durch Verbindungen der allgemeinen Formel **1** gelöst werden, in denen der Rest $R^7$ nicht Hydroxy bedeutet.

Dementsprechend zielt die vorliegende Erfindung auf Verbindungen der allgemeinen Formel **1**

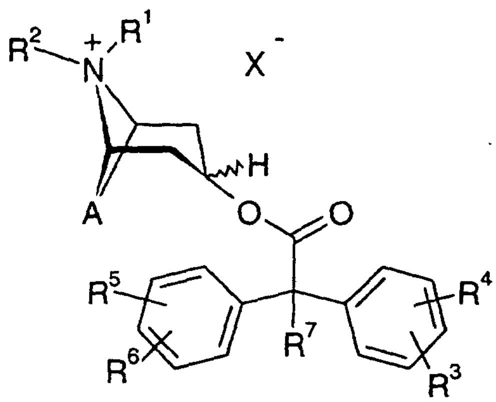

**1**

worin

A ein zweibindiger Rest ausgewählt aus der Gruppe bestehend aus

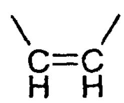 und 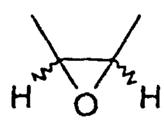 ;

$X^-$ ein einfach negativ geladenes Anion bevorzugt ausgewählt aus der Gruppe Chlorid, Bromid, Methylsulfat, 4-Toluolsulfonat und Methansulfonat, bevorzugt Bromid,

$R^1$ und $R^2$ gleich oder verschieden ein Rest ausgewählt aus der Gruppe bestehend aus Methyl, Ethyl, n-Propyl und iso-Propyl, der gegebenenfalls durch Hydroxy oder Fluor substituiert sein kann, bevorzugt unsubstituiertes Methyl;

$R^3$, $R^4$, $R^5$ und $R^6$, gleich oder verschieden, Wasserstoff, Methyl, Ethyl, Methyloxy, Ethyloxy, Hydroxy, Fluor, Chlor, Brom, CN, $CF_3$ oder $NO_2$;

$R^7$ Methyl, Ethyl, Methyloxy, Ethyloxy, $-CH_2-F$, $-CH_2-CH_2-F$, $-O-CH_2-F$, $-O-CH_2-CH_2-F$, $-CH_2-OH$, $-CH_2-CH_2-OH$, $CF_3$, $-CH_2-OMe$, $-CH_2-CH_2-OMe$, $-CH_2-OEt$, $-CH_2-CH_2-OEt$, -O-COMe, -O-CO-Et, $-O-COCF_3$, $-O-COCF_3$, Fluor, Chlor oder Brom, bedeuten.

Besonders bevorzugt sind Verbindungen der allgemeinen Formel **1**, worin

A ein zweibindiger Rest ausgewählt aus der Gruppe bestehend aus

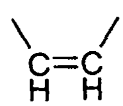 und 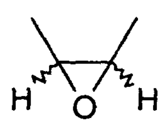 ;

$X^-$ ein einfach negativ geladenes Anion ausgewählt aus der Gruppe Chlorid, Bromid und Methan-

sulfonat, bevorzugt Bromid;

$R^1$ und $R^2$ gleich oder verschieden ein Rest ausgewählt aus Methyl und Ethyl, der gegebenenfalls durch Hydroxy oder Fluor substituiert sein kann, bevorzugt unsubstituiertes Methyl;

$R^3$, $R^4$, $R^5$ und $R^6$, gleich oder verschieden, Wasserstoff, Methyl, Ethyl, Methyloxy, Ethyloxy, Hydroxy, Fluor, Chlor oder Brom;

$R^7$ Methyl, Ethyl, Methyloxy, Ethyloxy, $CF_3$, oder Fluor, bedeuten.

Erfindungsgemäß bevorzugt sind Verbindungen der allgemeinen Formel **1**, worin

A ein zweibindiger Rest ausgewählt aus der Gruppe bestehend aus

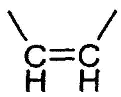 und 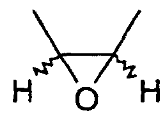 ;

$X^-$ Bromid;

$R^1$ und $R^2$ gleich oder verschieden ein Rest ausgewählt aus Methyl und Ethyl, bevorzugt Methyl;

$R^3$, $R^4$, $R^5$ und $R^6$, gleich oder verschieden, Wasserstoff, Methyl, Methyloxy, Chlor oder Fluor;

$R^7$ Methyl oder Fluor, bedeuten.

Erfindungsgemäß von besonderer Bedeutung sind Verbindungen der allgemeinen Formel **1**, worin

A ein zweibindiger Rest ausgewählt aus der Gruppe bestehend aus

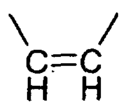 und 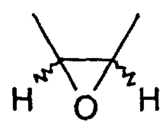 ;

$X^-$ Bromid;

$R^1$ und $R^2$ gleich oder verschieden Methyl oder Ethyl, bevorzugt Methyl;

$R^3$, $R^4$, $R^5$ und $R^6$, gleich oder verschieden, Wasserstoff oder Fluor, bevorzugt Wasserstoff;

$R^7$ Methyl oder Fluor, bevorzugt Methyl oder Fluor, besonders bevorzugt Methyl, bedeuten.

Gegenstand der Erfindung sind die jeweiligen Verbindungen der Formel **1** gegebenenfalls in Form der einzelnen optischen Isomeren, Mischungen der einzelnen Enantiomeren oder Racemate.

In den Verbindungen der allgemeinen Formel **1** können die Reste $R^3$, $R^4$, $R^5$ und $R^6$, sofern sie nicht Wasserstoff bedeuten, jeweils ortho, meta oder para bezüglich der Verknüpfung zur "-C- $R^7$"-Gruppe angeordnet sein. Sofern keiner der Reste $R^3$, $R^4$, $R^5$ und $R^6$ Wasserstoff bedeutet, sind $R^3$und $R^5$ bevorzugt in para-Position und $R^4$ und $R^6$ bevorzugt in ortho- oder meta-Position, besonders bevorzugt in meta-Position verknüpft. Sofern einer der Reste $R^3$ und $R^4$ und einer der Reste $R^5$ und $R^6$ Wasserstoff bedeutet, ist der jeweils andere Rest bevorzugt in meta- oder para-Position, besonders bevorzugt in para-Position verknüpft. Sofern keiner der Reste $R^3$, $R^4$, $R^5$ und $R^6$ Wasserstoff bedeutet sind erfindungsgemäß die Verbindungen der allgemeinen Formel **1** besonders bevorzugt, in denen die Reste $R^3$, $R^4$, $R^5$ und $R^6$ dieselbe Bedeutung aufweisen.

Von besonderer Bedeutung sind erfindungsgemäß die Verbindungen der allgemeinen Formel **1**, in denen der Ester-substituent am Stickstoff-bicylus α-konfiguriert ist Diese Verbindungen entsprechen der allgemeinen Formel **1-α**

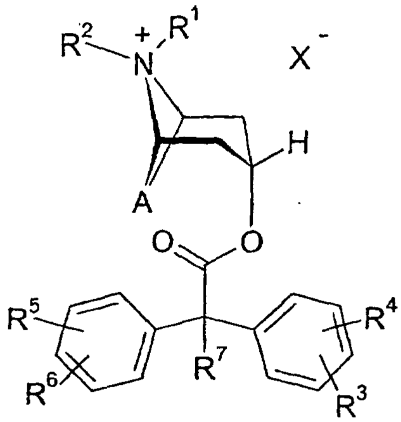

1-α.

Die nachfolgenden Verbindungen sind erfindungsgemäß von besonderer Bedeutung:

- 2,2-Diphenylpropionsäuretropenolester-methobromid;
- 2,2-Diphenylpropionsäurescopinester-methobromid;
- 2-Fluor-2,2-Diphenylessigsäurescopinester-methobromid;
- 2-Fluor-2,2-Diphenylessigsäuretropenolester-methobromid;

Als Alkylgruppen werden, soweit nicht anders angegeben, verzweigte und unverzweigte Alkylgruppen mit 1 bis 4 Kohlenstoffatomen bezeichnet. Beispielsweise werden genannt: Methyl, Ethyl, Propyl oder Butyl. Zur Bezeichnung der Gruppen Methyl, Ethyl, Propyl oder auch Butyl werden gegebenenfalls auch die Abkürzungen Me, Et, Prop oder Bu verwendet. Sofern nicht anders beschrieben, umfassen die Definitionen Propyl und Butyl alle denkbaren isomeren Formen der jeweiligen Reste. So umfaßt beispielsweise Propyl n-Propyl und iso-Propyl, Butyl umfaßt iso-Butyl, sec. Butyl und tert.-Butyl etc.

Als Alkylengruppen werden, soweit nicht anders angegeben, verzweigte und unverzweigte zweibindige Alkylbrücken mit 1 bis 4 Kohlenstoffatomen bezeichnet. Beispielsweise werden genannt: Methylen, Ethylen, Propylen oder Butylen.

Als Alkylen-Halogen-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch ein Halogen substituiert sind. Dementsprechend werden als Alkylen-OH-Gruppen, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch ein Hydroxy substituiert sind.

Als Alkyloxygruppen werden, soweit nicht anders angegeben, verzweigte und unverzweigte Alkylgruppen mit 1 bis 4 Kohlenstoffatomen bezeichnet, die über ein Sauerstoffatom verknüpft sind. Beispielsweise werden genannt: Methylox, Ethyloxy, Propyloxy oder Butyloxy. Zur Bezeichnung der Gruppen Methyloxy, Ethyloxy, Propyloxy oder auch Butyloxy werden gegebenenfalls auch die Abkürzungen MeO-, EtO-, PropO- oder BuO- verwendet. Sofern nicht anders beschrieben, umfassen die Definitionen Propyloxy und Butyloxy alle denkbaren isomeren Formen der jeweiligen Reste. So umfaßt beispielsweise Propyloxy n-Propyloxy und iso-Propyloxy, Butyloxy umfaßt iso-Butyloxy, sec. Butyloxy und tert.-Butyloxy etc. Gegebenenfalls wird im Rahmen der vorliegenden Erfindung statt der Bezeichnung Alkyloxy auch die Bezeichnung Alkoxy verwendet. Zur Bezeichnung der Gruppen Methyloxy, Ethyloxy, Propyloxy oder auch Butyloxy gelangen dementsprechend gegebenenfalls auch die Ausdrücke Methoxy, Ethoxy, Propoxy oder Butoxy zur Anwendung.

Als Alkylen-alkyloxy-Gruppen werden, soweit nicht anders angegeben, verzweigte und unverzweigte zweibindige Alkylbrücken mit 1 bis 4 Kohlenstoffatomen bezeichnet, die ein-, zwei- oder dreifach, bevorzugt einfach durch eine Alkyloxygruppe substituiert sind.

Als -O-CO-Alkylgruppen werden, soweit nicht anders angegeben, verzweigte und unverzweigte Alkylgruppen mit 1 bis 4 Kohlenstoffatomen bezeichnet, die über eine Estergruppe verknüpft sind. Dabei sind die Alkylgruppen direkt an den Carbonylkohlenstoff der Estergruppe gebunden. In analoger Art und Weise ist die Bezeichnung -O-CO-Alkyl-Halogen-gruppe zu verstehen. Die Gruppe -O-CO-$CF_3$ steht für Trifluoracetat.

Halogen steht im Rahmen der vorliegenden Erfindung für Fluor, Chlor, Brom oder Jod. Sofern nicht gegenteilig angegeben, gelten Fluor und Brom als bevorzugte Halogene. Die Gruppe CO bezeichnet eine Carbonylgruppe.

Die Herstellung der erfindungsgemäßen Verbindungen kann, wie nachstehend erläutert, zum Teil in Analogie zu im Stand der Technik bereits bekannten Vorgehensweisen erfolgen (Schema 1). Die Carbonsäurederivate der Formel **3** sind im Stand der Technik bekannt oder können nach im Stand der Technik bekannten Syntheseverfahren erhalten werden. Sind lediglich geeignet substituierte Carbonsäuren im Stand der Technik bekannt, können die Verbindungen der Formel **3** auch direkt aus diesen durch Säure- oder Basen-katalysierte Veresterung mit den entsprechenden Alkoholen oder durch Halogenierung mit den entsprechenden Halogenierungsreagentien erhalten werden.

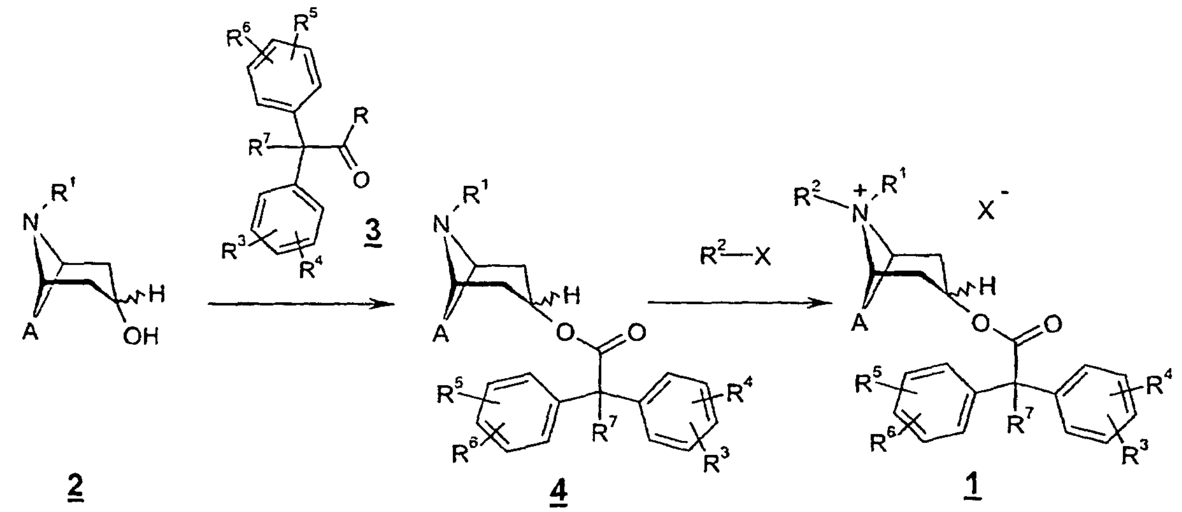


3

2 4 1

Schema 1:

Ausgehend von den Verbindungen der Formel **2** gelingt der Zugang zu den Estern der allgemeinen Formel **4** durch Umsetzung mit den Carbonsäurederivaten der Formel **3**, in denen R beispielsweise für Chlor oder einen $C_1$-$C_4$-Alkyloxyrest steht. Im Falle von R gleich $C_1$-$C_4$-Alkyloxy kann diese Umsetzung beispielsweise in einer Natriumschmelze bei erhöhter Temperatur, bevorzugt bei ca. 50-150°C, besonders bevorzugt bei etwa 90-100°C bei niedrigem Druck, bevorzugt bei unter 500mbar, besonders bevorzugt bei unter 75mbar durchgeführt werden. Alternativ dazu können statt der Derivate **3**, in denen R $C_1$-$C_4$-Alkyloxy bedeutet auch die entsprechenden Säurechloride (R gleich Cl) eingesetzt werden.

Die so erhaltenen Verbindungen der Formel **4** lassen sich durch Umsetzung mit den Verbindungen $R^2$-X, in denen R2 und X die vorstehend genannten Bedeutungen haben können, in die Zielverbindungen der Formel **1** überführen. Auch die Durchführung dieses Syntheseschritts kann in Analogie zu den in der WO 92/16528 offenbarten Synthesebeispielen erfolgen.

Alternativ zu der in Schema 1 dargestellten Vorgehensweise zur Synthese der Verbindungen der Formel **4** lassen sich die Derivate **4,** in denen der Stickstoffbicyclus ein Scopin-Derivat darstellt, durch Oxidation (Epoxidierung) von Verbindungen der Formel **4** erhalten, in denen der Stickstoffbicyclus ein Tropenyl-Rest ist. Hierzu kann erfindungsgemäß wie folgt vorgegangen werden.

Die Verbindung **4**, in der A für -CH=CH- steht, wird in einem polaren organischen Lösemittel, bevorzugt in einem Lösemittel ausgewählt aus der Gruppe N-Methyl-2-pyrrolidon (NMP), Dimethylacetamid und Dimethylformamid, bevorzugt Dimethylformamid suspendiert und anschließend erwärmt auf eine Temperatur von ca. 30-90°C, vorzugsweise 40-70°C. Anschließend wird ein geeignetes Oxidationsmittel zugegeben und bei konstanter Temperatur 2 bis 8 Stunden, bevorzugt 3 bis 6 Stunden gerührt. Als Oxidationsmittel kommt bevorzugt Vanadiumpentoxid im Gemisch mit $H_2O_2$, besonders bevorzugt $H_2O_2$-Harnstoff komplex in Kombination mit Vanadiumpentoxid zur Anwendung. Die Aufarbeitung erfolgt auf üblichem Wege. Die Reinigung der Produkte kann je nach Kristallisationsneigung durch Kristallisation oder Chromatographie erfolgen.

Alternativ dazu sind die Verbindungen der Formel **4,** in denen $R^7$ Halogen bedeutet auch auf dem in Schema 2 dargestellten Wege zugänglich.

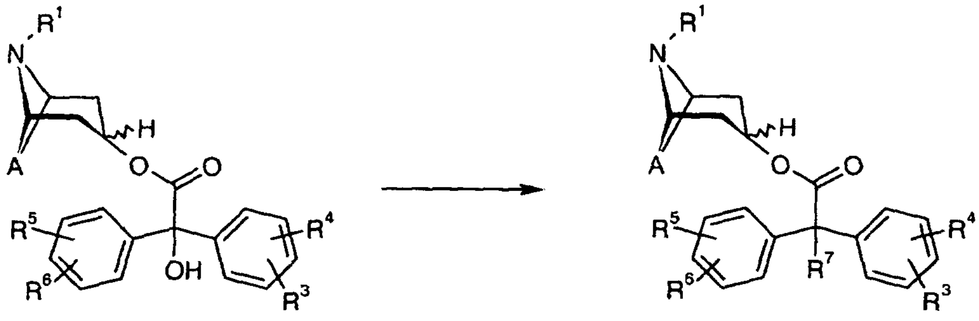

5 4 (mit $R^7$ Halogen)

Schema 2:

Hierzu werden die Benzilsäureester der Formel **5** unter Verwendung geeigneter Halogenierungsreagentien in die Verbindungen **4**, in denen $R^7$ Halogen bedeutet. Die Durchführung der nach Schema 2 durchzuführenden Halogenierungsreaktionen ist im Stand der Technik hinreichend bekannt.

Die Benzilsäureester der Formel **5** sind nach oder on Analogie zu den im Stand der Technik bekannten Verfahren zugänglich (siehe z.B. WO 92/16528).

Die nachstehend beschriebenen Synthesebeispiele dienen der weitergehenden Illustration der vorliegenden Erfindung. Sie sind allerdings nur als exemplarische Vorgehensweisen zur weitergehenden Erläuterung der Erfindung zu verstehen, ohne selbige auf den nachfolgend exemplarisch beschriebenen Gegenstand zu beschränken.

**Beispiel 1:** 2,2-Diphenylpropionsäurescopinester-Methobromid :

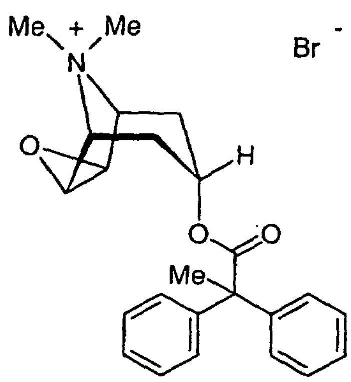

**1.1.:** 2,2-Diphenylpropionsäurechlorid **3a:**

Zu einer Suspension aus 25,0 g (0,11 mol) 2,2-Diphenylpropionsäure, 100 ml Dichlormethan und 4 Tropfen Dimethylformamid.werden bei 20° C 52,08g (0,33 mol) Oxalylchlorid langsam zugetropft. Es wird 1 h bei 20°C und 0,5 h bei 50° C gerührt.

Das Lösungsmittel wird abdestilliert und der verbleibende Rückstand ohne weitergehende Reinigung in die nächste Stufe eingesetzt.

**1.2.:** 2,2-Diphenylpropionsäurescopinester **4a:**

Der aus Stufe 1.1. erhaltene Rückstand wird in 100 ml Dichlormethan gelöst und bei 40° C tropfenweise mit einer Lösung aus 51,45 g (0,33 mol) Scopin in 200 ml Dichlormethan versetzt. Die entstandene Suspension wird 24 h bei 40° C gerührt, anschließend der entstandene Niederschlag abgesaugt und das Filtrat zunächst mit Wasser, dann wässriger Salzsäure sauer extrahiert. Die vereinigten wässrigen Phasen werden mit wässriger Natriumcarbonatlösung alkalisch gestellt, mit Dichlormethan extrahiert, die organische Phase über $Na_2SO_4$ getrocknet, zur Trockene einge-

dampft und aus dem Rückstand das Hydrochlorid gefällt. Die Reinigung erfolgt durch Umkristallisation aus Acetonitril.
Ausbeute: 20,85 g (= 47 % d. Th.)
DC: Rf-Wert: 0,24 (Laufmittel: sek. Butanol/Ameisensäure/Wasser 75:15:10);
Smp.: 203-204°C.

**1.3:** 2,2-Diphenylpropionsäurescopinester-Methobromid :

11,98 g (0,033 mol) **4a**, 210 ml Acetonitril, 70 ml Dichlormethan und 20, 16 g (0,1 mol) 46,92 % iges Brommethan in Acetonitril werden bei 20°C zusammengegeben und 3 Tage stehen gelassen. Die Lösung wird zur Trockene eingedampft und der Rückstand aus Isopropanol umkristallisiert.
Ausbeute: 11,34 g (= 75 % d. Th); Smp.: 208-209°C.
$C_{24}H_{28}NO_3xBr$ (458,4);

| | | | | |
|---|---|---|---|---|
| Elementaranalyse | berechnet | C (62,89) | H (6,16) | N (3,06) |
| | gefunden. | C (62,85) | H (6,12) | N (3,07). |

**Beispiel 2:** 2-Fluor-2,2-diphenylessigsäurescopinester-Methobromid :

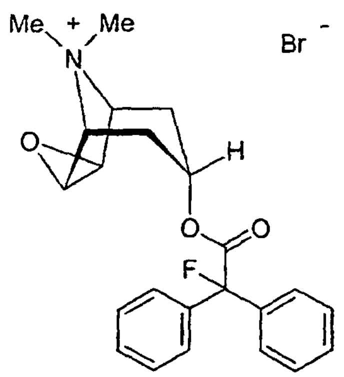

**2.1:** Benzilsäurescopinester **5a:**

Die Herstellung des Benzilsäurescopinesters ist im Stand der Technik bekannt. Sie wird in der WO 92/16528 beschrieben.

**2.2:** 2-Fluor-2,2-Diphenylessigsäurescopinester **4b:**

2,66 g (0,02 mol) Dimethylaminoschwefeltrifluorid werden in 10 ml Dichlormethan auf 0°C gekühlt und eine Lösung aus 5,48 g (0,015 mol) Benzilsäurescopinester **5a** in 100 ml Dichlormethan zugetropft. Anschließend wird 30 min bei 0°C und 30 min bei 20°C nachgerührt. Unter Kühlung wird die Lösung mit Wasser versetzt, $NaHCO_3$ zugegeben (bis pH 7-8) und die organische Phase abgetrennt. Die Wasserphase wird mit Dichlormethan extrahiert, die vereinten organischen Phasen mit Wasser gewaschen, über $Na_2SO_4$ getrocknet und zur Trockene eingedampft.
Aus dem Rückstand wird das Hydrochlorid gefällt und aus Acetonitril umkristallisiert.
Ausbeute: 6,90 g (= 85 % d. Th.)
Smp.: 227°-230° C.

**2.3:** 2-Fluor-2,2-Diphenylessigsäurescopinester-Methobromid :

2,88 g (0,0078 mol) der freien Base des Benzilsäurescopinesters werden analog zur Durchführung unter Stufe 1.3 umgesetzt. Die Reinigung erfolgt durch Umkristallisation aus Isopropanol. Ausbeute: 2,62 g (= 73 % d. Th.)
DC: Rf-Wert: 0,31 (Laufmittel analog Stufe 1.2); Smp.: 130-134°C.

**Beispiel 3:** 2,2-Diphenylpropionsäuretropenolester-Methobromid :

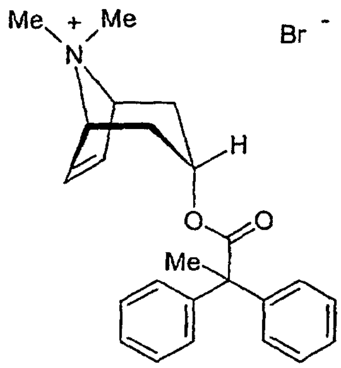

**3.1.:** 2,2-Diphenylpropionsäuremethylester **3b:.**

In die Suspension von 50,8 g (0,225 mol) 2,2-Diphenylpropionsäure und 200 ml Acetonitril werden bei 20°C 37,60 g (0,247 mol) DBU zugetropft. Zu der entstandenen Lösung werden 70,10 g (0,494 mol) Methyliodid innerhalb 30 min zugetropft. Anschließend wird über Nacht bei 20° C gerührt. Das Lösungsmittel wird eingedampft, der Rückstand mit Diethylether/Wasser extrahiert, die organische Phase mit Wasser gewaschen, über $Na_2SO_4$ getrocknet und zur Trockene eingedampft. Ausbeute: 48,29 g viskoser Rückstand 32 (= 89 % d. Th.).

**3.2:** 2,2-Diphenylpropionsäuretropenolester **4c:**

4,80 g (0,02 mol) 2,2-Diphenylpropionsäuremethylester **3b,** 2,78 g (0,02 mol) Tropenol und 0,046 g Natrium werden als Schmelze bei 75 mbar 4 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit Wasser gewaschen, über $MgSO_4$ getrocknet und zur Trockene eingedampft.
Aus dem Rückstand wird **4c** als Hydrochlorid gefällt und dieses aus Aceton umkristallisiert.
Ausbeute: 5,13 g (= 67 % d. Th.);
DC: Rf-Wert: 0,28 (Laufmittel: sek. Butanol/Ameisensäure/Wasser 75:15:10);
Smp.: 134-135°C.

**3.3:** 2,2-Diphenylpropionsäuretropenolester-Methobromid :

2,20 g (0,006 mol) **4c** werden analog zu Beispiel 1, Stufe 1.3 umgesetzt. Die entstandenen Kristalle werden abesaugt, mit Dichlormethan gewaschen, getrocknet und anschließend aus Methanol/Diethylether umkristallisiert.
Ausbeute: 1,84 g (=66 % d. Th.)
DC: Rf-Wert: 0,11 (Laufmittel analog Stufe 1.2); Smp.: 222-223°C. $C_{24}H_{28}NO_2xBr$ (442,4);

| | | | | |
|---|---|---|---|---|
| Elementaranalyse | berechnet | C (65,16) | H (6,38) | N (3,17) |
| | gefunden. | C (65,45) | H (6,29) | N (3,16). |

**Beispiel 4:** 2-Fluor-2,2-bis(3,4-difluorphenyl)essigsäuretropenolester-Methobromid :

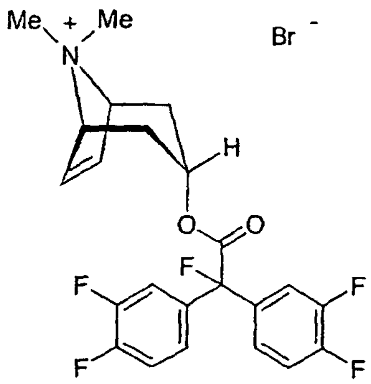

**4.1.:**3,3',4,4'-Tetrafluorbenzilsäureethylester **3c:.**

Die Herstellung des Grignard-Reagenzes erfolgt aus 2,24 g (0,092 mol) Magnesiumspäne, einigen Körnchen Jod und 17,80 g (0,092 mol) 1-Brom-3,4-difluor-benzol in 100 ml THF bei 50° C. Nach beendeter Zugabe des Halogenids wird noch eine Stunde nachgerührt. Das so erhaltene Grignard-Reagenz wird zu 18,81 g (0,088 mol) 3,4-Diffuorphenylglyoxylsäureethylester in 80 ml THF bei 10°-15° C tropfenweise zugesetzt und die erhaltene Mischung 2 Stunden bei 5° C gerührt.
Die weiße Suspension wird zur Aufarbeitung auf Eis/Schwefelsäure gegossen, mit Ethylacetat extrahiert, die organische Phase mit Wasser gewaschen, über $MgSO_4$ getrocknet und zur Trockene eingedampft. Die Reinigung des Rohprodukts erfolgt durch Säulenchromatographie (Laufmittel: Toluol).
Ausbeute: 10,80 g Öl 1 (= 38 % d. Th.)

**4.2.:** 3,3',4,4'-Tetrafluorbenzilsäuretropenolester **5b:**

4,27 g (0,013 mol) 3,3',4,4'-Tetrafluorbenzilsäureethylester **3c**, 1,81 g (0,013 mol) Tropenol und 0,03 g Natrium werden als Schmelze bei 75 mbar 4 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit Wasser gewaschen, über $MgSO_4$ getrocknet und zur Trockene eingedampft.
Der verbleibende Rückstand wird mit Diethylether/Petrolether 1:9 versetzt, abgesaugt und gewaschen. Ausbeute: 2,50 g (= 46 % d. Th.);
DC: Rf-Wert: 0,29 (Laufmittel: sek. Butanol/Ameisensäure/Wasser 75:15:10);
Smp.: 147°-148°C.

**4.3:** 2-Fluor-2,2-bis(3,4-difluorphenyl)essigsäuretropenolester **4d:**

2,66 g (0,012 mol) Bis-(2-methoxyethyl)-aminosulfurtrifluorid wurden in 10 ml Dichlormethan vorgelegt und innerhalb von 20 Minuten bei 15°-20° C mit einer Lösung aus 0,01 mol **5b** in 65 ml Dichlormethan tropfenweise versetzt. Es wird 20 h bei Raumtemperatur gerührt, abgekühlt auf 0° C und vorsichtig mit 80 ml Wasser unter gutem Rühren versetzt. Anschließend wird vorsichtig mit wässeriger $NaHCO_3$-Lösung auf pH 8 eingestellt, die organische Phase abgetrennt, die Wasserphase erneut mit Dichlormethan extrahiert, die vereinten organischen Phasen mit Wasser gewaschen, über $MgSO_4$ getrocknet und zur Trockene eingedampft. Es wird das Hydrochlorid gefällt und aus Acetonitril/Diethylether umkristallisiert.
Ausbeute: 2,60 g weiße Kristalle (= 57 % d. Th.)
Smp.: 233° C

**4.4:** 2-Fluor-2,2-bis(3,4-difluorphenyl)essigsäuretropenolester-Methobromid :

2,20 g (0,0052 mol) **4d** werden analog zu Beispiel 1, Stufe 1.3 umgesetzt. Die entstandenen Kristalle werden abesaugt, mit Dichlormethan gewaschen, getrocknet und anschließend aus Methanol/Diethylether umkristallisiert.

Ausbeute: 1,95 g (=72 % d. Th.)
DC: Rf-Wert: 0,17 (Laufmittel: n-Butanol/Wasser/Ameisensäure(konz.)/Aceton/Dichlormethan 36:15:15:15:5); Smp.: 247°C.
$C_{23}H_{21}F_5NO_2xBr$ (518,3);

| | | | | |
|---|---|---|---|---|
| Elementaranalyse | berechnet | C (53,30) | H (4,08) | N (2,70) |
| | gefunden. | C (53,22) | H (4,19) | N (2,69). |

**Beispiel 5:** 2,2-Diphenylpropionsäurescopinester-Ethylbromid :

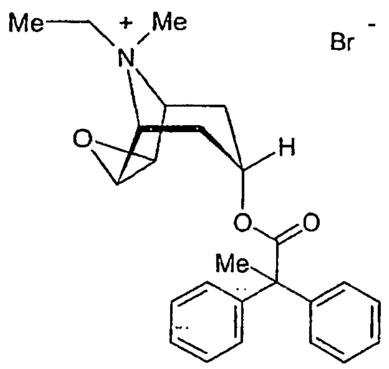

1,81 g (0,005 mol) **4a** , 35 ml Acetonitril und 1,64 g (0,015 mol) Ethylbromid werden bei 20°C zusammengegeben und 3 Tage stehen gelassen. Die Lösung wird zur Trockene eingedampft und der Rückstand aus Ethanol umkristallisiert.
Ausbeute: 1,38 g (= 58 % d. Th); Smp.: 208-209°C.
DC: Rf-Wert: 0,33 (Laufmittel analog Stufe 1.2); Smp.: 210-211°C.
$C_{25}H30NO_3xBr$ (472,42);

| | | | | |
|---|---|---|---|---|
| Elementaranalyse | berechnet | C (63,56) | H (6,40) | N (2,96) |
| | gefunden. | C (63,49) | H (6,24) | N (2,88). |

**Beispiel 6:** 2-Fluor-2,2-bis(3,4-difluorphenyl)essigsäurescopinester-Methobromid :

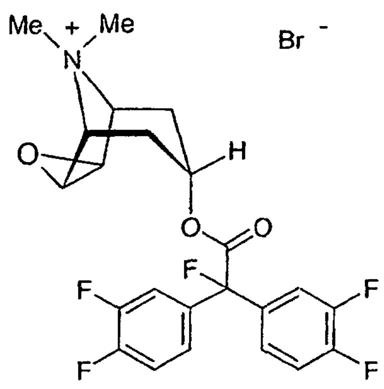

**6.1.:** 3,3',4,4'-Tetrafluorbenzilsäurescopinester **5c:**

3,61 g (0,011 mol) 3,3',4,4'-Tetrafluorbenzilsäureethylester **3c**, 1,71 g (0,011 mol) Scopin und 0,03 g Natrium werden als Schmelze bei 75 mbar 4 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit Wasser gewaschen, über $MgSO_4$ getrocknet und zur Trockene eingedampft.
Der verbleibende Rückstand wird mit Diethylether/Petrolether 1:9 versetzt, abgesaugt und gewaschen. Ausbeute: 1,75 g (= 36 % d. Th.);
Smp.: 178-179°C.

**6.2:** 2-Fluor-2,2-bis(3,4-difluorphenyl)essigsäurescopinester **4e:**

0,6 ml (0,0033 mol) Bis-(2-methoxyethyl)-aminosulfurtrifluorid werden mit 1,2 g (0,0028 mol) **5c** in Analogie zu Beispiel 4, Stufe 4.3 umgesetzt.
Ausbeute: 1,15 g farbloses Öl (= 95 % d. Th.)

**6.3:** 2-Fluor-2,2-bis(3,4-difluorphenyl)essigsäurescopinester-Methobromid :

1,15 g (0,0026 mol) **4e** und 1,5 g (0.0079 mol) 50%-ige Methylbromidlösung werden analog zu Beispiel 1, Stufe 1.3 umgesetzt. Die entstandenen Kristalle werden abesaugt, mit Dichlormethan gewaschen, getrocknet und anschließend aus Aceton umkristallisiert.
Ausbeute: 0,88 g (=63 % d. Th.)
DC: Rf-Wert: 0,27 (Laufmittel: n-Butanol/Wasser/Ameisensäure(konz.)/Aceton/Dichlormethan 36:15:15:15:5); Smp.: 212°C.
$C_{23}H_{21}F_5NO_3xBr$ (535,33);

**Beispiel 7:** 2-Fluor-2,2-bis(4-fluorphenyl)essigsäuretropenolester-Methobromid :

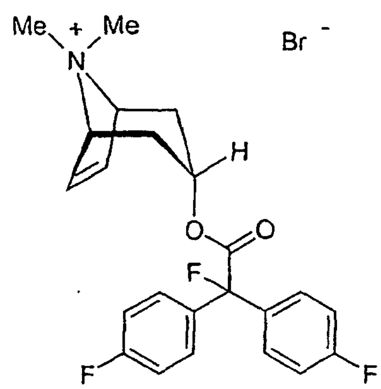

**7.1.:** 4,4'-Difluorbenzilsäuremethylester **3d:.**

7.1.1.: 4,4'-Difluorbenzilsäure:

Zu einer Lösung aus 49,99 g (1,25 mol) NaOH-Plätzchen in 300 ml Wasser wird bei ca. 100° C eine Lösung aus 24,62 g (0,1 mol) 4,4'-Difluorbenzil in 250 ml Dioxan zugetropft und 2 h gerührt. Das Dioxan wird größtenteils abdestilliert und die verbleibende wässrige Lösung mit Dichlormethan extrahiert. Beim Ansäuern der wässrigen Lösung mit Schwefelsäure fällt ein Niederschlag aus, der abgesaugt, gewaschen und getrocknet wird. Das Filtrat wird mit Dichlormethan. extrahiert, die organische Phase über $Na_2SO_4$ getrocknet und zur Trockene eingedampft. Ausbeute: 25,01 g (= 95 % d. Th.); Smp.: 133°-136° C

7.1.2.: 4,4'-Difluorbenzilsäuremethylester:

Zu frisch hergestellter Natriumethanolatlösung aus 2,17 g (0,095 mol) Natrium und 200 ml Ethanol werden bei 20° C 25,0 g (0,095 mol) 4,4'-Difluorbenzilsäure zugegeben und 3 h gerührt. Die Lösung wird zur Trockene eingedampft, der Rückstand in DMF gelöst, bei 20° C tropfenweise mit 22,57 g (0,16 mol) Methyljodid versetzt und 24 h gerührt. Die Aufarbeitung und Reinigung erfolgt in Analogie zu Verbindung **3b.** Ausbeute: 21,06 g 11 (= 80 % d. Th.)

**7.2.:** 4,4'-Difluorbenzilsäuretropenolester **5d:**

11,13 g (0,04 mol) 4,4'-Difluorbenzilsäuremethylester **3d** und 5,57 g (0,04 mol) Tropenol werden mit 0,09 g Natrium werden in Analogie zu Beispiel 3, Stufe 3.2 umgesetzt. Das Produkt wird aus Acetonitril umkristallisiert.
Ausbeute: 10,43 g (= 62 % d. Th.);
Smp.: 233-235°C.

**7.3:** 2-Fluor-2,2-bis(4-fluorphenyl)-essigsäuretropenolester **4f:**

2,94 g (0,013 mol) Bis-(2-methoxyethyl)-aminosulfurtrifluorid werden mit 3,85 g (0,01 mol) **5d** in Analogie zu Beispiel 4, Stufe 4.3 in 100 ml Dichlormethan umgesetzt. Das Produkt wird in Form seines Hydrochlorids aus Acetonitril umkristallisiert.
Ausbeute: 2,93 g (= 69 % d. Th.)

**7.4:** 2-Fluor-2,2-bis(4-fluorphenyl)-essigsäuretropenolester-Methobromid :

2,6 g (0,0067 mol) **4f** und 1,9 g (0.0079 mol) 50%-ige Methylbromidlösung werden analog zu Beispiel 1, Stufe 1.3 umgesetzt. Die entstandenen Kristalle werden abesaugt, mit Dichlormethan gewaschen, getrocknet und anschließend aus Methanol/Diethylether umkristallisiert.
Ausbeute: 2,82 g weiße Kristalle (=87 % d. Th.)
DC: Rf-Wert: 0,55 (Laufmittel: gemäß Beispiel 1, Stufe 1.2);
Smp.: 230-231°C.
$C_{23}H_{23}F_3NO_2xBr$ (482,34);

| | | | | |
|---|---|---|---|---|
| Elementaranalyse | berechnet | C (57,27) | H (4,81) | N (2,90) |
| | gefunden. | C (57,15) | H (4,84) | N (2,96). |

**Beispiel 8:** 2-Fluor-2,2-bis(4-fluorphenyl)essigsäurescopinester-Methobromid :

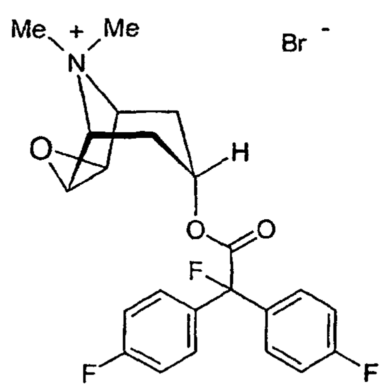

**8.1:** 4,4'-Difluorbenzilsäurescopinester **5e:**

4,22 g (0,01 mol) 4,4'-Difluorbenzilsäuretropenolester **5d** werden in 80 ml DMF suspendiert. Bei ca. 40°C Innentemperatur wird eine Lösung aus 2,57 g (0,0273 mol) $H_2O_2$-Harnstoff in 20 ml Wasser, sowie 0,2 g (0,0011 mol) Vanadium-(V)-oxid zugegeben und 4,5 h bei 60°C gerührt. Nach Abkühlen auf 20°C wird der entstandene Niederschlag

abgesaugt, das Filtrat mit 4 N Salzsäure auf pH 3 gestellt und mit $Na_2S_2O_5$ gelöst in Wasser versetzt. Die dadurch entstandene grüne Lösung wird zur Trockene eingedampft, der Rückstand mit Dichlormethan/Wasser extrahiert. Die saure Wasserphase wird mit $Na_2CO_3$ basisch gestellt, mit Dichlormethan extrahiert und die organische Phase über $Na_2SO_4$ getrocknet und konzentriert. Anschließend erfolgte die Zugabe von 0,5 ml Acetylchlorid bei ca. 15°C und 1,5 h Rühren. Nach Extraktion mit 0,1 N Salzsäure wird die Wasserphase basisch gestellt, mit Dichlormethan extahiert, die organische Phase über $Na_2SO_4$ getrocknet und zur Trockene eingedampft. Aus dem Rückstand wird das Hydrochlorid gefällt und aus Methanol/Diethylether umkristallisiert.
Ausbeute: 3,61 g weiße Kristalle (= 78 % d. Th.);
Smp.: 243-244°C.

**8.2:** 2-Fluor-2,2-bis(4-fluorphenyl)-essigsäurescopinester **4g:**

1,48 g (0,0067 mol) Bis-(2-methoxyethyl)-aminosulfurtrifluorid werden mit 2,0 g (0,005 mol) **5e** in Analogie zu Beispiel 4, Stufe 4.3 in 80 ml Dichlormethan umgesetzt. Das Produkt wird in Form seines Hydrochlorids aus Ethanol umkristallisiert.
Ausbeute: 2,07 g (= 94 % d. Th.); Smp.: 238-239°C.

**8.3:** 2-Fluor-2,2-bis(4-fluorphenyl)-essigsäurescopinester-Methobromid :

1,6 g (0,004 mol) **4g** und 1,14 g (0.0079 mol) 50%-ige Methylbromidlösung werden analog zu Beispiel 1, Stufe 1.3 umgesetzt. Die entstandenen Kristalle werden abesaugt, mit Dichlormethan gewaschen, getrocknet und anschließend aus Acetonitril umkristallisiert.
Ausbeute: 1,65 g weiße Kristalle (=61 % d. Th.)
DC: Rf-Wert: 0,25 (Laufmittel: gemäß Beispiel 1, Stufe 1.2);
Smp.: 213-214°C.
$C_{23}H_{23}F_3NO_3xBr$ (498,34);

| | | | | |
|---|---|---|---|---|
| Elementaranalyse | berechnet | C (55,43) | H (4,65) | N (2,81) |
| | gefunden. | C (54,46) | H (4,67) | N (2,80). |

**Beispiel 9:** 2-Fluor-2,2-diphenylessigsäuretropenolester-Methobromid :

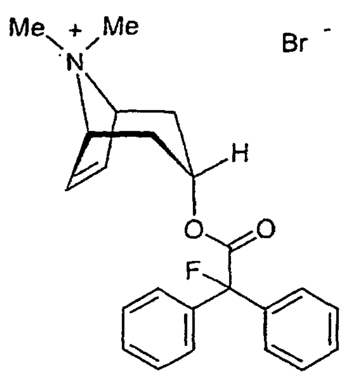

**9.1.:** Benzilsäuretropenolester **5f:.**

Der Benzilsäuretropenolester sowie Verfahren zu dessen Herstellung sind aus der WO 92/16528 bekannt.

**9.2:** 2-Fluor-2,2-diphenylessigsäuretropenolester **4h:**

15,86 ml (0,086 mol) Bis-(2-methoxyethyl)-aminosulfurtrifluorid werden mit 25 g (0,072 mol) **5f** in Analogie zu Beispiel 4, Stufe 4.3 in 480 ml Chloroform umgesetzt. Das Produkt wird in Form seines Hydrochlorids aus Aceton

umkristallisiert.
Ausbeute: 18,6 g weiße Kristalle (= 67 % d. Th.);
Smp.: 181-182°C;

**9.3:** 2-Fluor-2,2-diphenyl-essigsäuretropenolester-Methobromid :

11,12 g (0,032 mol) **4h** und 18,23 g (0.096 mol) 50%-ige Methylbromidlösung werden analog zu Beispiel 1, Stufe 1.3 umgesetzt. Die entstandenen Kristalle werden aus Acetonitril umkristallisiert.
Ausbeute: 11,91 g weiße Kristalle (=83 % d. Th.)
DC: Rf-Wert: 0,4 (Laufmittel: gemäß Beispiel 4, Stufe 4.4);
Smp.: 238-239°C.
$C_{23}H_{25}FNO_2xBr$ (446,36);

| | | | | |
|---|---|---|---|---|
| Elementaranalyse | berechnet | C (61,89) | H (5,65) | N (3,14) |
| | gefunden. | C (62,04) | H (5,62) | N (3,17). |

**Beispiel 10:** 2-Fluor-2,2-(3-chlorphenyl)essigsäuretropenolester-Methobromid :

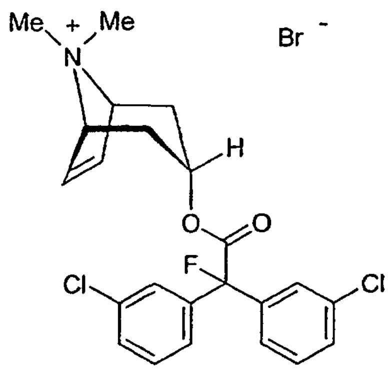

**10.1.:**3.3'-Dichlorbenzilsäuremethylester **3e:.**

10.1.1.: 3,3'-Dichlorbenzil:

100 ml Ethanol werden bei Raumtemperatur vorgelegt und 50,0 g (0,356 mol) 3-Chlorbenzaldehyd und 4,54 g (0,018 mol) 3-Ethyl-5-(2-hydroxyethyl)-4-methylthiazoliumbromid zugegeben. Anschließend werden 10,7 g (0,11 mol) Triethylamin zugetropft. Es wird 3 h unter Rückfluß gekocht und zur Trockene eingedampft. Der Rückstand wird in Ethylacetat aufgenommen und mit Wasser, Natriumpyrosulfit in Wasser und Na2CO3-Lösung extrahiert. Nach Trocknen über MgSO4 wird bis zur Trockene eingedampft. Das erhaltene Produkt wird aus Isopropanol und Petrolether umkristallisiert.
Ausbeute: 13,2 g weiße Kristalle (= 13% d. Th.); Smp.: 69-70°C.

13,0 g des so erhattenen Acyloins werden in 460 ml Acetonitril bei RT gelöst, 0,0867 g Vanadium-(V)-oxytrichlorid zugegeben und Sauerstoff eingeleitet. Nach 1,5 h wird die Lösung zur Trockene eingedampft, mit Ethylacetat und Wasser, sowie Na2CO3-Lösung extrahiert, über MgSO4 getrocknet und zur Trockene eingedampft. Der verbleibende Rückstand wird mit Petrolether/Ethylacetat 95:5 ausgerührt.
Ausbeute: 12,59 g gelbe Kristalle (= 97% d. Th.); Smp.: 116-117°C.

10.1.2.: 3,3'-Dichlorbenzilsäure:

51,45 g (1.286 mol) Natriumhydroxid in 1000 ml Wasser werden unter gutem Rühren im kochenden Wasserbad vorgelegt und eine Lösung aus 28,5 g (0,102 mol) 3,3'-Dichlorbenzil in 700 ml Dioxan zugetropft und anschließende 1 h nachgerührt.
Nach Abkühlung wird das Dioxan eingedampft, der Rückstand mit Wasser verdünnt und mit Diethylether extrahiert.

Die organische Phase wird sauer gestellt , mit Dichlormethan extrahiert, über MgSO4 getrocknet, zur Trockene eingedampft.
Ausbeute: 32,7 g (= 71% d. Th.).

10.1.3.: 3,3'-Dichlorbenzilsäuremethylester:

Aus 100 ml Ethanol und 1,97 g (0,0855 mol) Natrium wird eine Natriumethanolatlösung hergestellt, zu der 26,6 g (0,0855 mol) 3,3'-Dichlorbenzilsäure in 50 ml Ethanol getropft wird. anschließend wird 4 h bei Raumtemeperatur gerührt. Nach Abdestillieren des Lösungsmittels wird der Rückstand in 150 ml DMF gelöst und 24,27 g (0,171 mol) Methyljodid zugetropft, anschließend weitere 24 h gerührt. Unter Eiskühlung werden 300 ml Wasser und 200 ml Diethylether zugetropft, die Phasen getrennt, die Wasserphase mit Diethylether extrahiert, anschließend die organischen Phasen mit Na2CO3-Lösung gewaschen und mit Wasser neutral geschüttelt. Nach Trocknen über Na2SO4 wird zur Trockene eingedampft. Ausbeute: 22,91 g gelbes Öl (= 82% d. Th.).

**10.2.:** 3,3'-Dichlorbenzilsäuretropenolester **5g:**

22,9 g (0,074 mol) 3,3'-Dichlorbenzilsäuremethylester **3e,** 15,37 g (0,11 mol) Tropenol und 0,17 g Natrium werden als Schmelze bei 75 mbar 4 h auf kochendem Wasserbad unter gelegentlichem Schütteln erhitzt. Nach Abkühlung werden die Natriumreste mit Acetonitril aufgelöst, die Lösung zur Trockene eingedampft und der Rückstand mit Dichlormethan/Wasser extrahiert. Die organische Phase wird mit Wasser gewaschen, über $MgSO_4$ getrocknet und zur Trockene eingedampft. Das Produkt wird in Form seines HYdrochlorids aus Acetonitril umkristallisiert.
Ausbeute: 16,83 g weiße Kristalle (= 50 % d. Th.);
Smp.: 184-185°C.

**10.3:** 2-Fluor-2,2-bis(3-chlorphenyl)essigsäuretropenolester **4i:**

1,48 g (0,0067 mol) Bis-(2-methoxyethyl)-aminosulfurtrifluorid werden in 10 ml Dichlormethan vorgelegt und innerhalb von 20 Minuten bei 15°-20° C mit einer Lösung aus 2,09 g **5g** in 65 ml Dichlormethan tropfenweise versetzt. Es wird 20 h bei Raumtemperatur gerührt, abgekühlt auf 0° C und vorsichtig mit 80 ml Wasser unter gutem Rühren versetzt. Anschließend wird vorsichtig mit wässeriger $NaHCO_3$-Lösung auf pH 8 eingestellt, die organische Phase abgetrennt, die Wasserphase erneut mit Dichlormethan extrahiert, die vereinten organischen Phasen mit Wasser gewaschen, über $MgSO_4$ getrocknet und zur Trockene eingedampft. Es wird das Hydrochlorid gefällt und aus Acetonitril/Diethylether umkristallisiert.
Ausbeute: 1,20 g weiße Kristalle (= 53 % d. Th.)
Smp.: 136-137° C

**10.4:** 2-Fluor-2,2-bis(3-chlorphenyl)essigsäuretropenolester-Methobromid :

1,0 g (0,002 mol) **4h** werden analog zu Beispiel 1, Stufe 1.3 umgesetzt. Die entstandenen Kristalle werden abesaugt, mit Dichlormethan gewaschen, getrocknet und anschließend aus Methanol/Diethylether umkristallisiert.
Ausbeute: 0,82 g weiße Kristalle (=80 % d. Th.)
DC: Rf-Wert: 0,14 (Laufmittel: n-Butanol/Wasser/Ameisensäure(koriz.)/Aceton/Dichlormethan 36:15:15:15:5); Smp.: 180-181°C.
$C_{23}H_{23}Cl_2FNO_2xBr$ (515,25);

Wie gefunden wurde, zeichnen sich die Verbindungen der allgemeinen Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als Anticholinergikum bevorzugt zur Anwendung gelangen können.
Dies sind beispielsweise die Therapie von Asthma oder COPD (chronic obstructive pulmonary disease = chronisch obstruktive Lungenerkrankung). Die Verbindungen der allgemeinen Formel **1** können ferner zur Behandlung vagal bedingter Sinusbradykardien und zur Behandlung von Herz-Rhythmus-Störungen zum Einsatz gelangen. Generell lassen sich die erfindungsgemäßen Verbindungen ferner zur Behandlung von Spasmen beispielsweise im Gastrointestinaltrakt mit therapeutischem Nutzen einsetzen. Sie können ferner bei der Behandlung von Spasmen in harnableitenden Wegen sowie beispielsweise bei Menstruationsbeschwerden zum Einsatz gelangen.
Von den vorstehend beispielhaft aufgeführten Indikationsgebieten, kommt der Therapie von Asthma und COPD mittels der erfindungsgemäßen Verbindungen der Formel **1** eine besondere Bedeutung zu.
Die Verbindungen der allgemeinen Formel **1** können allein oder in Kombination mit anderen erfindungsgemäßen Wirkstoffen der Formel **1** zur Anwendung gelangen. Gegebenenfalls können die Verbindungen der allgemeinen

Formel **1** auch in Kombination mit weiteren pharmakologisch aktiven Wirkstoffen eingesetzt werden. Es handelt sich hierbei insbesondere um Betamimetica, Antiallergika, PAF-Antagonisten, Leukotrien-Antagonisten und Corticosteroiden, sowie Wirkstoffkombinationen davon.

Als Beispiel für Betamimetika, die erfindungsgemäß mit den Verbindungen der Formel **1** als Kombination zum Einsatz kommen können, seien genannt Verbindungen, die ausgewählt sind aus der Gruppe bestehend aus Bambuterol, Bitolterol, Carbuterol, Clenbuterol, Fenoterol, Formoterol, Hexoprenalin, Ibuterol, Pirbuterol, Procaterol, Reproterol, Salmeterol, Sulfonterol, Terbutalin, Tolubuterol, 4-Hydroxy-7-[2-{[2-{[3-(2-phenylethoxy)propyl]sulfonyl}ethyl]-amino}ethyl]-2(3H)-benzothiazolon, 1-(2-Fluoro-4-hydroxyphenyl)-2-[4-(1-benzimidazolyl)-2-methyl-2-butylamino]ethanol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-nbutyloxyphenyl)-2-methyl-2-propylamino]ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-Hydroxy-8-(1-hydroxy-2-isopropylaminobutyl)-2H-1,4-benzoxazin-3-(4H)-on, 1-(4-Amino-3-chloro-5-trifluormethylphenyl)-2-*tert*.butylamino)ethanol und 1-(4-Ethoxycarbonylamino-3-cyano-5-fluorophenyl)-2-(*tert*.butylamino)ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Besonders bevorzugt gelangen als Betamimetika solche Wirkstoffe in Kombination mit den erfindungsgemäßen Verbindungen der Formel **1** zur Anwendung, die ausgewählt sind aus der Gruppe bestehend aus Fenoterol, Formoterol, Salmeterol, 1-[3-(4-Methoxybenzyl-amino)-4-hydroxyphenyl]-2-[4-(1-benzimidazolyl)-2-methyl-2-butylamino]ethanol, 1-[2H-5-Hydroxy-3-oxo-4H-1,4-benzoxazin-8-yl]-2-[3-(4-N,N-dimethylaminophenyl)-2-methyl-2-propylamino]ethanol, 1-[2H-5-Hydroxy-3-oxo-4H-1,4-benzoxazin-8-yl]-2-[3-(4-methoxyphenyl)-2-methyl-2-propylamino]ethanol, 1-[2H-5-Hydroxy-3-oxo-4H-1,4-benzoxazin-8-yl]-2-[3-(4-n-butyloxyphenyl)-2-methyl-2-propylamino] ethanol, 1-[2H-5-Hydroxy-3-oxo-4H-1,4-benzoxazin-8-yl]-2-{4-[3-(4-methoxyphenyl)-1,2,4-triazol-3-yl]-2-methyl-2-buty (amino}ethanol, gegebenenfalls in Form ihrer Racemate, ihrer Enantiomere, ihrer Diastereomere, sowie gegebenenfalls ihrer pharmakologisch unbedenklichen Säureadditionssalze und Hydrate. Von den vorstehend genannten Betamimetika kommt hierbei den Verbindungen Formoterol und Salmeterol gegebenenfalls in Form ihrer Racemate, ihrer Enantiomere, ihrer Diastereomere, sowie gegebenenfalls ihrer pharmakologisch unbedenklichen Säureadditionssalze und Hydrate besondere Bedeutung zu.

Erfindungsgemäß bevorzugt sind die Säureadditionssalze der Betamimetika ausgewählt aus der Gruppe bestehend aus Hydrochlorid, Hydrobromid, Sulfat, Phosphat, Fumarat Methansulfonat und Xinafoat. Besonders bevorzugt sind die Salze im Falle des Salmeterols ausgewählt aus Hydrochlorid, Sulfat und Xinafoat, von denen die Sulfate und Xinafoate besonders bevorzugt sind. Erfindungsgemäß von herausragender Bedeutung sind Salmeterol x ½ $H_2SO_4$ und Salmeterolxinafoat. Besonders bevorzugt sind die Salze im Falle des Formoterols ausgewählt aus Hydrochlorid, Sulfat und Fumarat, von denen das Hydrochlorid und Fumarat besonders bevorzugt sind. Erfindungsgemäß von herausragender Bedeutung ist Formoterolfumarat.

Im Rahmen der vorliegenden Erfindung werden unter Corticosteroiden, die gegebenenfalls in Kombination mit den Verbindungen der Formel **1** zum Einsatz gelangen können, Verbindungen verstanden, die ausgewählt sind aus der Gruppe bestehend aus Flunisolide, Beclomethasone, Triamcinolone, Budesonid, Fluticasone, Mometasone, Ciclesonide, Rofleponide, GW 215864, KSR 592, ST-126 und Dexametasone. Bevorzugt sind im Rahmen der vorliegenden Erfindung die Corticosteroide ausgewählt aus der Gruppe bestehend aus Flunisolide, Beclomethasone, Triamcinolone, Budesonid, Fluticasone, Mometasone, Ciclesonide und Dexametasone, wobei hier dem Budesonid, Fluticasone, Mometasone und Ciclesonide, insbesondere dem Budesonid und dem Fluticason eine besondere Bedeutung zukommt. Gegebenfalls wird im Rahmen der vorliegenden Patentanmeldung statt der Bezeichnung Corticosteroide auch nur die Bezeichnung Steroide verwendet. Eine Bezugnahme auf Steroide schließt im Rahmen der vorliegenden Erfindung eine Bezugnahme auf Salze oder Derivate, die von den Steroiden gebildet werden können, mit ein. Als mögliche Salze oder Derivate werden beispielsweise genannt: Natriumsalze, Sulfobenzoate, Phosphate, Isonicotinate, Acetate, Propionate, Dihydrogenphosphate, Palmitate, Pivalate oder Furoate. Gegebenenfalls können die Corticosteroide auch in Form ihrer Hydrate vorliegen.

Im Rahmen der vorliegenden Erfindung werden unter Dopamin-Agonisten, die gegebenenfalls in Kombination mit den Verbindungen der Formel **1** zum Einsatz gelangen können, Verbindungen verstanden, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan. Bevorzugt werden im Rahmen der vorliegenden Erfindung Dopamin-Agonisten als Kombinationspartner mit den Verbindungen der Formel **1** eingesetzt, die ausgewählt sind aus der Gruppe bestehend aus Pramipexol, Talipexol und Viozan, wobei Pramipexol eine besondere Bedeutung zukommt. Eine Bezugnahme auf die vorstehend genannten Dopamin-Agonisten schließt im Rahmen der vorliegenden Erfindung eine Bezugnahme auf deren gegebenenfalls existierende pharmakologisch verträgliche Säureadditionssalze und gegebenenfalls deren Hydrate ein. Unter den physiologisch verträglichen Säureadditionssalzen, die von den vorstehend genannten Dopaminagonisten gebildet werden können, werden beispielsweise pharmazeutisch verträgliche Salze verstanden, die ausgewählt aus den Salzen der Salzsäure; Bromwasserstoffsäure, Schwefelsäure, Phosphorsäure, Methansulfonsäure, Essigsäure, Fumarsäure, Bernsteinsäure, Milchsäure, Zitronensäure, Weinsäure und Maleinsäure sind.

Als Beispiel für Antiallergika, die erfindungsgemäß mit den Verbindungen der Formel **1** als Kombination zum Einsatz kommen können, seien genannt Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin. Bevorzugte Antiallergika, die im Rahmen der vorliegenden Erfindung in Kombination mit den erfindungsgemäßen Verbindungen der Formel **1** zum Einsatz gelangen können, sind ausgewählt aus der Gruppe bestehend aus Epinastin, Cetirizin, Azelastin, Fexofenadin, Levocabastin, Loratadin, Ebastin, Desloratidin und Mizolastin wobei Epinastin und Desloratidin besonders bevorzugt sind. Eine Bezugnahme auf die vorstehend genannten Antiallergika schließt im Rahmen der vorliegenden Erfindung eine Bezugnahme auf deren gegebenenfalls existierende pharmakologisch verträgliche Säureadditionssalze ein.

Als Beispiel für PAF-Antagonisten, die erfindungsgemäß mit den Verbindungen der Formel **1** als Kombination zum Einsatz kommen können seien genannt 4-(2-Chlorphenyl)-9-methyl-2-[3(4-morpholinyl)-3-propanon-1-yl]-6Hthieno-[3,2-f][1,2,4]triazolo[4,3-a][1,4]diazepin, 6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin.

Werden die Verbindungen der Formel **1** in Kombination mit anderen Wirkstoffen eingesetzt, ist von den vorstehend genannten Verbindungsklassen die Kombination mit Steroiden oder Betamimetika besonders bevorzugt. Der Kombination mit Betamimetika, insbesondere mit langwirksamen Betamimetika kommt dabei eine besondere Bedeutung zu. Als besonders bevorzugt ist die Kombination der erfindungsgemäßen Verbindungen der Formel **1** mit Salmeterol oder Formoterol anzusehen, wobei die Kombination mit Formoterol höchst bevorzugt ist.

Geeignete Anwendungsformen zur Applikation der Verbindungen der Formel **1** sind beispielsweise Tabletten, Kapseln, Zäpfchen, Lösungen etc.

Erfindungsgemäß von besonderer Bedeutung ist (insbesondere bei der Behandlung von Asthma oder COPD) die inhalative Applikation der erfindungsgemäßen Verbindungen. Der Anteil der pharmazeutisch wirksamen Verbindung (en) sollte jeweils im Bereich von 0,05 bis 90 Gew.-%, bevorzugt 0,1 bis 50 Gew.-% der Gesamtzusammensetzung liegen. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Lösungen werden in üblicher Weise, z.B. unter Zusatz von Isotonantien, Konservierungsmitteln, wie p-Hydroxybenzoaten, oder Stabilisatoren, wie Alkalisalzen der Ethylendiamintetraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z. B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzukker) Emulgiermittel (z.B. Lignin, Sufitablaugen, Methylcellulose, Stärke und Polyvinylpyrrolidon) und Gleitmittel (z.B.

Magnesiumstearat, Talkum, Stearinsäure und Natriumlaurylsulfat) erwähnt.

Die Applikation erfolgt in üblicher Weise, bei der Therapie von Asthma oder COPD vorzugsweise inhalativ. Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Die Dosierung der erfindungsgemäßen Verbindungen ist naturgemäß stark von der Applikationsart und der zu therapierenden Erkrankung abhängig. Bei inhalativer Applikation zeichnen sich die Verbindungen der Formel **1** bereits bei Dosen im µg-Bereich durch eine hohe Wirksamkeit aus. Auch oberhalb des µg-Bereichs, lassen sich die Verbindungen der Formel **1** sinnvoll einsetzen. Die Dosierung kann dann beispielsweise auch im Grammbereich liegen. Insbesondere bei nicht inhalativer Applikation können die erfindungsgemäßen Verbindungen mit höherer Dosierung appliziert werden (beispielsweise, aber nicht limitierend im Bereich von 1 bis 1000mg).

Die nachfolgenden Formulierungsbeipiele illustrieren die vorliegende Erfindung ohne sie jedoch in ihrem Umfang zu beschränken:

Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| D) | Dosieraerosol | |
|---|---|---|
| | Wirkstoff | 0,005 |
| | Sorbitantrioleat | 0,1 |
| | Monofluortrichlormethan und Difluordichlormethan 2 : 3 | ad 100 |

Die Suspension wird in einen üblichen Aerosolbehälter mit Dosierventil gefüllt. Pro Betätigung werden vorzugsweise 50 µl Suspension abgegeben. Der Wirkstoff kann gewünschtenfalls auch höher dosiert werden (z.B. 0.02 Gew.-%).

| E) | Lösungen (in mg/100ml) | |
|---|---|---|
| | Wirkstoff | 333.3 mg |
| | Formoterolfumarat | 333.3 mg |
| | Benzalkoniumchlorid | 10.0 mg |
| | EDTA | 50.0 mg |
| | HCl (1n) | ad pH 3.4 |

Diese Lösung kann in üblicher Art und Weise hergestellt werden.

| F) | Inhalationpulver | |
|---|---|---|
| | Wirkstoff | 6 µg |
| | Formoterolfumarat | 6 µg |
| | Lactose Monohydrat | ad 25 mg |

Die Herstellung des Inhaltionspulvers erfolgt in üblicher Art und Weise durch Mischen der einzelnen Bestandteile.

| G) | Inhalationpulver | |
|---|---|---|
| | Wirkstoff | 10 µg |
| | Lactose Monohydrat | ad 5 mg |

Die Herstellung des Inhaltionspulvers erfolgt in üblicher Art und Weise durch Mischen der einzelnen Bestandteile.

## Patentansprüche

**1.** Verbindungen der allgemeinen Formel <u>1</u>

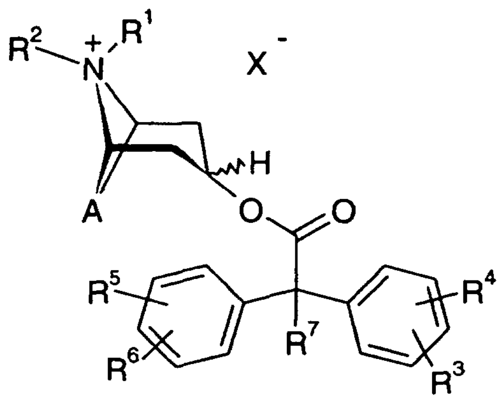

1

worin

A ein zweibindiger Rest ausgewählt aus der Gruppe bestehend aus

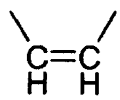 und 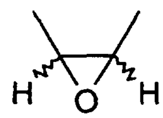 ;

$X^-$ ein einfach negativ geladenes Anion, bevorzugt ausgewählt aus der Gruppe Chlorid, Bromid, Methylsulfat, 4-Toluolsulfonat und Methansulfonat;

$R^1$ und $R^2$ gleich oder verschieden ein Rest ausgewählt aus der Gruppe bestehend aus Methyl, Ethyl, n-Propyl und iso-Propyl, der gegebenenfalls durch Hydroxy oder Fluor substituiert sein kann;

$R^3$, $R^4$, $R^5$ und $R^6$, gleich oder verschieden, Wasserstoff, Methyl, Ethyl, Methyloxy, Ethyloxy, Hydroxy, Fluor, Chlor, Brom, CN, $CF_3$ oder $NO_2$;

$R^7$ Methyl, Ethyl, Methyloxy, Ethyloxy, $-CH_2-F$, $-CH_2-CH_2-F$, $-O-CH_2-F$, $-O-CH_2-CH_2-F$, $-CH_2-OH$, $-CH_2-CH_2-OH$, $CF_3$, $-CH_2-OMe$, $-CH_2-CH_2-OMe$, $-CH_2-OEt$, $-CH_2-CH_2-OEt$, -O-COMe, -O-COEt, $-O-COCF_3$, $-O-COCF_3$, Fluor, Chlor oder Brom, bedeuten,

gegebenenfalls in Form der einzelnen optischen Isomeren, Mischungen der einzelnen Enantiomeren oder Racemate.

**2.** Verbindungen der allgemeinen Formel 1, gemäß Anspruch **1,** worin

A ein zweibindiger Rest ausgewählt aus der Gruppe bestehend aus

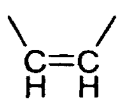 und 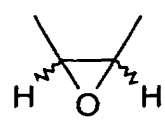 ;

$X^-$ ein einfach negativ geladenes Anion ausgewählt aus der Gruppe Chlorid, Bromid und Methansulfonat;

$R^1$ und $R^2$ gleich oder verschieden ein Rest ausgewählt aus Methyl und Ethyl, der gegebenenfalls durch Hydroxy oder Fluor substituiert sein kann;

$R^3$, $R^4$, $R^5$ und $R^6$, gleich oder verschieden, Wasserstoff, Methyl, Ethyl, Methyloxy, Ethyloxy, Hydroxy, Fluor, Chlor oder Brom;

$R^7$ Methyl, Ethyl, Methyloxy, Ethyloxy, $CF_3$, oder Fluor, bedeuten,

gegebenenfalls in Form der einzelnen optischen Isomeren, Mischungen der einzelnen Enantiomeren oder Racemate.

**3.** Verbindungen der allgemeinen Formel **1**, gemäß einem der Ansprüche 1 oder 2, worin

A ein zweibindiger Rest ausgewählt aus der Gruppe bestehend aus

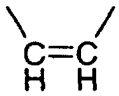 und 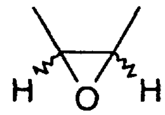 ;

$X^-$ Bromid;
$R^1$ und $R^2$ gleich oder verschieden ein Rest ausgewählt aus Methyl und Ethyl;
$R^3$, $R^4$, $R^5$ und $R^6$, gleich oder verschieden, Wasserstoff, Methyl, Methyloxy, Chlor oder Fluor;
$R^7$ Methyl oder Fluor, bedeuten,

gegebenenfalls in Form der einzelnen optischen Isomeren, Mischungen der einzelnen Enantiomeren oder Racemate.

**4.** Verbindungen der allgemeinen Formel **1,** gemäß einem der Ansprüche 1 bis 3, worin

A ein zweibindiger Rest ausgewählt aus der Gruppe bestehend aus

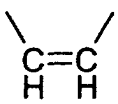 und 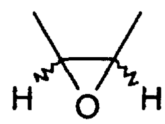 ;

$X^-$ Bromid;
$R^1$ und $R^2$ gleich oder verschieden Methyl oder Ethyl;
$R^3$, $R^4$, $R^5$ und $R^6$, gleich oder verschieden, Wasserstoff oder Fluor;
$R^7$ Methyl oder Fluor, bedeuten,

gegebenenfalls in Form der einzelnen optischen Isomeren, Mischungen der einzelnen Enantiomeren oder Racemate.

**5.** Verwendung einer Verbindung der allgemeinen Formel **1** gemäß einem der Ansprüche 1 bis 4 als Arzneimittel.

**6.** Verwendung einer Verbindung der allgemeinen Formel **1** gemäß einem der Ansprüche 1 bis 4 zur Herstellung eines Arzneimittels zur Behandlung von Asthma, COPD, vagal bedingter Sinusbradykardien, Herz-Rhythmus-Störungen, Spasmen im Gastrointestinaltrakt, Spasmen in harnableitenden Wegen und Menstruationsbeschwerden.

**7.** Verwendung einer Verbindung der allgemeinen Formel **1** gemäß Anspruch 6 zur Herstellung eines Arzneimittels zur Behandlung von Asthma oder COPD.

**8.** Pharmazeutische Zubereitungen, enthaltend als Wirkstoff eine oder mehrere Verbindungen der allgemeinen Formel **1** gemäß einem der Ansprüche 1 bis 4 oder deren physiologisch verträgliche Salze gegebenenfalls in Kombination mit üblichen Hilfs- und/oder Trägerstoffen.

**9.** Pharmazeutische Zubereitungen nach Anspruch 8, **dadurch gekennzeichnet daß** diese neben einer oder mehrerer der Verbindungen der Formel **1** ferner wenigstens einen weiteren Wirkstoff enthalten, der ausgewählt ist aus der Gruppe der Betamimetica, Antiallergika, PAF-Antagonisten, Leukotrien-Antagonisten und Steroide.

**10.** Verfahren zur Herstellung einer Verbindung der allgemeinen Formel **1**

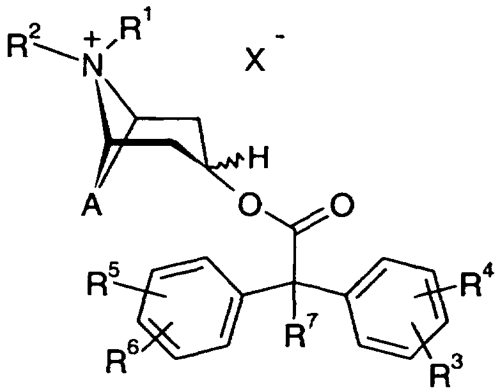

**1**

worin A, $X^-$ und die Reste $R^1$, $R^2$, $R^3$, $R^4$, $R^5$, $R^6$ und $R^7$, die in den Ansprüchen 1 bis 4 genannten Bedeutungen haben können, **dadurch gekennzeichnet, daß** man in einem ersten Schritt eine Verbindung der allgemeinen Formel **3**

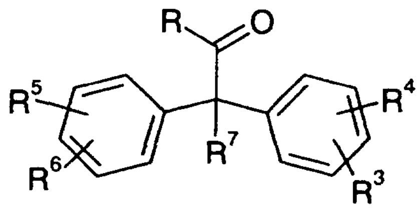

**3**

worin die Reste $R^3$, $R^4$, $R^5$, $R^6$ und $R^7$, die in den Ansprüchen 1 bis 4 genannten Bedeutungen haben können und R für Chlor oder einen $C_1$-$C_4$-Alkyloxy steht mit einer Verbindung der Formel **2**

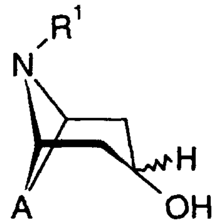

**2**

worin A und $R^1$ die in den Ansprüchen 1 bis 4 genannten Bedeutungen haben können zu einer Verbindung der Formel **4**

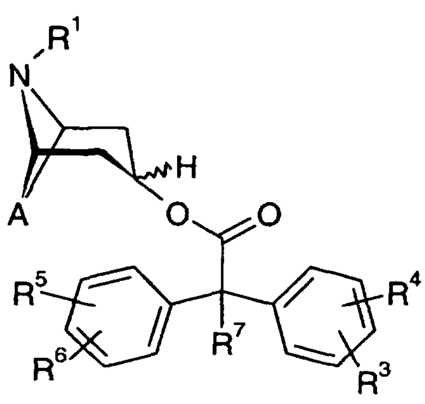

**4**

worin A und die Reste $R^1$, $R^3$, $R^4$, $R^5$, $R^6$ und $R^7$, die in den Ansprüchen 1 bis 4 genannten Bedeutungen haben können, umsetzt und diese anschließend durch Umsetzung mit einer Verbindung $R^2$-X, worin $R^2$ und X die in den Ansprüchen 1 bis 4 genannten Bedeutungen haben können zu einer Verbindung der Formel **1** quarternisiert.

## Claims

**1.** Compounds of general formula **1**

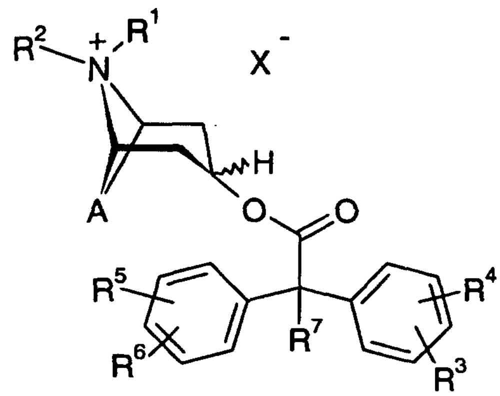

**1**

wherein

| | |
|---|---|
| A | denotes a double-bonded group selected from among |

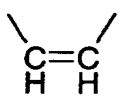 und 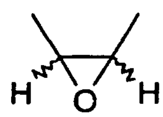 ;

| | |
|---|---|
| $X^-$ | denotes an anion with a single negative charge preferably selected from among chloride, bromide, methylsulphate, 4-toluenesulphonate and methanesulphonate; |
| $R^1$ and $R^2$ | which may be identical or different denote a group selected from among methyl, ethyl, n-propyl and iso-propyl, which may optionally be substituted by hydroxy or fluorine; |
| $R^3$, $R^4$, $R^5$ and $R^6$, | which may be identical or different, denote hydrogen, methyl, ethyl, methyloxy, ethyloxy, hydroxy, fluorine, chlorine, bromine, CN, $CF_3$ or $NO_2$; |
| $R^7$ | denotes methyl, ethyl, methyloxy, ethyloxy, $-CH_2-F$, $-CH_2-CH_2-F$, $-O-CH_2-F$, $-O-CH_2-CH_2-F$, $-CH_2-OH$, $-CH_2-CH_2-OH$, $CF_3$, $-CH_2-OMe$, $-CH_2-CH_2-OMe$, $-CH_2-OEt$, $-CH_2-CH_2-OEt$, -O-COMe, -O-COEt, $-O-COCF_3$, $-O-COCF_3$, fluorine, chlorine or bromine, |

optionally in the form of the individual optical isomers, mixtures of the individual enantiomers or racemates thereof.

**2.** Compounds of general formula **1,** according to claim 1, wherein

| | |
|---|---|
| A | denotes a double-bonded group selected from among |

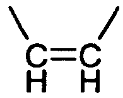 und 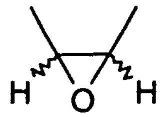 ;

$X^-$ denotes an anion with a single negative charge selected from among chloride, bromide and methanesulphonate;

$R^1$ and $R^2$ which may be identical or different denote a group selected from methyl and ethyl, which may optionally be substituted by hydroxy or fluorine;

$R^3$, $R^4$, $R^5$ and $R^6$, which may be identical or different, denote hydrogen, methyl, ethyl, methyloxy, ethyloxy, hydroxy, fluorine, chlorine or bromine;

$R^7$ denotes methyl, ethyl, methyloxy, ethyloxy, $CF_3$ or fluorine,

optionally in the form of the individual optical isomers, mixtures of the individual enantiomers or racemates thereof.

**3.** Compounds of general formula **1**, according to one of claims 1 and 2,
wherein

A denotes a double-bonded group selected from among

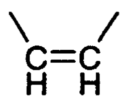 und 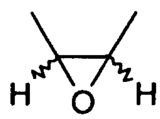 ;

$X^-$ denotes bromide;

$R^1$ and $R^2$ which may be identical or different denote a group selected from methyl and ethyl;

$R^3$, $R^4$, $R^5$ and $R^6$, which may be identical or different, denote hydrogen, methyl, methyloxy, chlorine or fluorine;

R7 denotes methyl or fluorine,

optionally in the form of the individual optical isomers, mixtures of the individual enantiomers or racemates thereof.

**4.** Compounds of general formula **1**, according to one of claims 1 to 3,
wherein

A denotes a double-bonded group selected from among

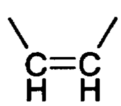 und 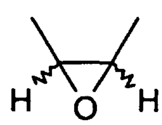 ;

$X^-$ denotes bromide;

$R^1$ and $R^2$ which may be identical or different denote methyl or ethyl;

$R^3$, $R^4$, $R^5$ and $R^6$, which may be identical or different, denote hydrogen or fluorine;

$R^7$ denotes methyl or fluorine,

optionally in the form of the individual optical isomers, mixtures of the individual enantiomers or racemates thereof.

5. Use of a compound of general formula **1** according to one of claims 1 to 4 as a medicament.

6. Use of a compound of general formula **1** according to one of claims 1 to 4 for preparing a medicament for the treatment of asthma, COPD, vagally induced sinus bradycardia, heart rhythm disorders, spasms in the gastrointestinal tract, spasms in the urinary tract and menstrual disorders.

7. Use of a compound of general formula **1** according to claim 6 for preparing a medicament for the treatment of asthma or COPD.

8. Pharmaceutical preparations containing as active substance one or more compounds of general formula **1** according to one of claims 1 to 4 or the physiologically acceptable salts thereof, optionally combined with conventional excipients and/or carriers.

9. Pharmaceutical preparations according to claim 8, **characterised in that** they contain, in addition to one or more of the compounds of formula **1**, at least one further active substance which is selected from among the betamimetics, antiallergic agents, PAF-antagonists, leukotriene-antagonists and steroids.

10. Process for preparing a compound of general formula **1**

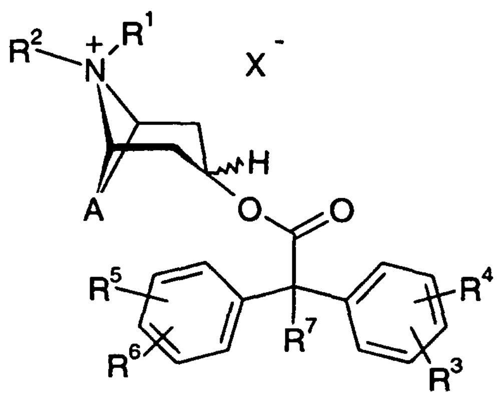

**1**

wherein A, $X^-$ and the groups $R^1$, $R^2$, $R^3$, $R^4$, $R^5$, $R^6$ and $R^7$ may have the meanings given in claims 1 to 4, **characterised in that** in a first step a compound of general formula **3**

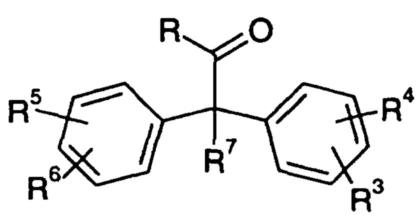

**3**

wherein the groups R3, $R^4$, $R^5$, $R^6$ and $R^7$ may have the meanings given in claims 1 to 4 and R denotes chlorine or a $C_1$-$C_4$-alkyloxy, is reacted with a compound of formula **2**

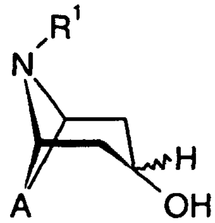

**2**

wherein A and $R^1$ may have the meanings given in claims 1 to 4, to obtain a compound of formula **4**

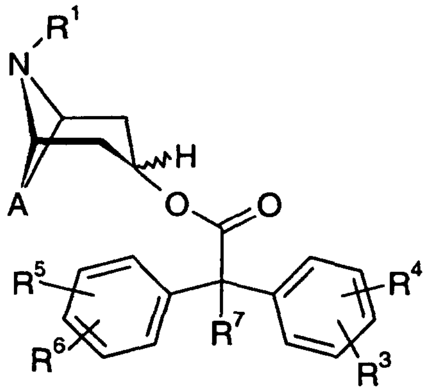

**4**

wherein A and the groups $R^1$, $R^3$, $R^4$, $R^5$, $R^6$ and $R^7$ may have the meanings given in claims 1 to 4, and this is then quaternised by reacting with a compound $R^2$-X, wherein $R^2$ and X may have the meanings given in claims 1 to 4 to obtain a compound of formula **1**.

## Revendications

**1.** Composés de formule générale 1

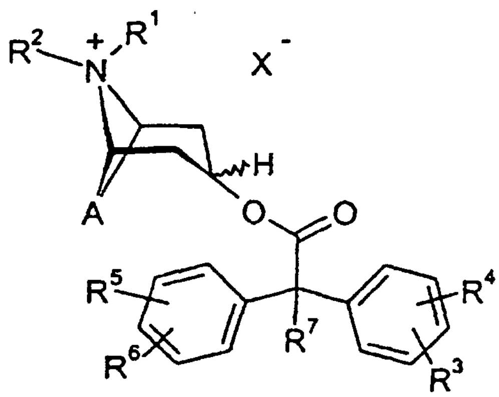

**1**

où

A représente un reste divalent choisi dans le groupe consistant en

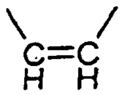 et 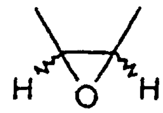 ;

$X^-$ représente un anion à une charge négative de préférence choisi dans le groupe chlorure, bromure, méthylsulfate, 4-toluènesulfonate et méthanesulfonate ; $R^1$ et $R^2$, identiques ou différents, représentent un reste choisi dans le groupe consistant en méthyle, éthyle, n-propyle et isopropyle, qui peut éventuellement être substitué par hydroxyle ou fluor ;
$R^3$, $R^4$, $R^5$ et $R^6$, identiques ou différents, représentent l'hydrogène, méthyle, éthyle, méthyloxy, éthyloxy, hydroxyle, le fluor, le chlore, le brome, CN, $CF_3$ ou $NO_2$ ;
$R^7$ représente méthyle, éthyle, méthyloxy, éthyloxy, $-CH_2-F$, $-CH_2-CH_2-F$, $-O-CH_2-F$, $-O-CH_2-CH_2-F$, $-CH_2-OH$, $-CH_2-CH_2-OH$, $-CF_3$, $-CH_2-OMe$, $-CH_2-CH_2-OMe$, $-CH_2-OEt$, $-CH_2-CH_2-OEt$, -O-COMe, -O-COEt, $-O-COCF_3$, $-O-COCF_3$, le fluor, le chlore ou le brome,
éventuellement sous forme des différents isomères optiques, de mélanges des différents énantiomères ou de racémates.

**2.** Composés de formule générale 1 selon la revendication 1
où
A représente un reste divalent choisi dans le groupe consistant en

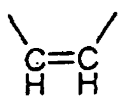 et 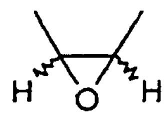 ;

X- représente un anion à une charge négative choisi dans le groupe chlorure, bromure et méthanesulfonate ;
$R^1$ et $R^2$, identiques ou différents, représentent un reste choisi parmi méthyle et éthyle, qui peut éventuellement être substitué par hydroxyle ou le fluor ;
$R^3$, $R^4$, $R^5$ et $R^6$, identiques ou différents, représentent l'hydrogène, méthyle, éthyle, méthyloxy, éthyloxy, hydroxyle, le fluor, le chlore ou le brome ;
$R^7$ représente méthyle, éthyle, méthyloxy, éthyloxy, $CF_3$ ou le fluor,
éventuellement sous forme des différents isomères optiques, de mélanges des différents énantiomères ou de racémates.

**3.** Composés de formule générale 1 selon l'une des revendications 1 ou 2
où
A représente un reste divalent choisi dans le groupe consistant en

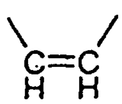 et 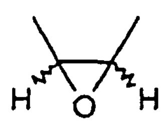 ;

$X^-$ représente le bromure ;
$R^1$ et $R^2$, identiques ou différents, représentent un reste choisi parmi méthyle et éthyle ;
$R^3$, $R^4$, $R^5$ et $R^6$, identiques ou différents, représentent l'hydrogène, méthyle, méthyloxy, le chlore ou le fluor ;
$R^7$ représente méthyle ou le fluor,
éventuellement sous forme des différents isomères optiques, de mélanges des différents énantiomères ou de racémates.

**4.** Composés de formule générale 1 selon l'une des revendications 1 à 3
où

A représente un reste divalent choisi dans le groupe consistant en

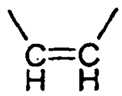

et

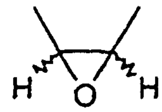

;

$X^-$ représente le bromure ;
$R^1$ et $R^2$, identiques ou différents, représentent méthyle ou éthyle ;
$R^3$, $R^4$, $R^5$ et $R^6$, identiques ou différents, représentent l'hydrogène ou le fluor ;
$R^7$ représente méthyle ou le fluor,
éventuellement sous forme des différents isomères optiques, de mélanges des différents énantiomères ou de racémates.

**5.** Utilisation d'un composé de formule générale 1 selon l'une des revendications 1 à 4 comme médicament.

**6.** Utilisation d'un composé de formule générale 1 selon l'une des revendications 1 à 4 pour la préparation d'un médicament pour le traitement de l'asthme, de la COPD, des bradycardies sinusales d'origine vagale, des troubles du rythme cardiaque, des spasmes dans les voies gastro-intestinales, des spasmes dans les voies urinaires et des troubles menstruels.

**7.** Utilisation d'un composé de formule générale 1 selon la revendication 6 pour la préparation d'un médicament pour le traitement de l'asthme ou de la COPD.

**8.** Préparations pharmaceutiques contenant comme principe actif un ou plusieurs composés de formule générale 1 selon l'une des revendications 1 à 4 ou leurs sels physiologiquement acceptables éventuellement en combinaison avec des adjuvants et/ou supports courants.

**9.** Préparations pharmaceutiques selon la revendication 8 **caractérisées en ce qu'**elles contiennent, outre un ou plusieurs des composés de formule 1, au moins un autre principe actif qui est choisi dans le groupe des bêtamimétiques, des anti-allergiques, des antagonistes de PAF, des antagonistes des leucotriènes et des stéroïdes.

**10.** Procédé de préparation d'un composé de formule générale 1

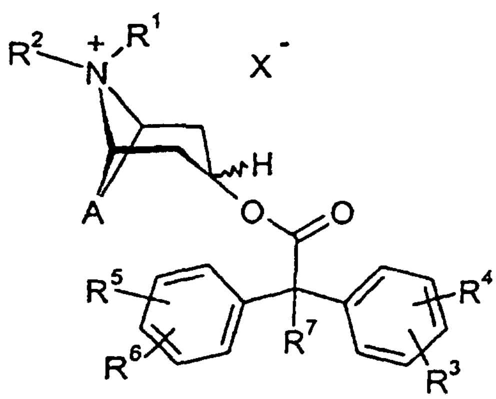

**1**

où A, $X^-$ et les restes $R^1$, $R^2$, $R^3$, $R^4$, $R^5$, $R^6$ et $R^7$ peuvent avoir les significations citées dans les revendications 1 à 4, **caractérisé en ce que**, dans une première étape, on convertit un composé de formule générale 3

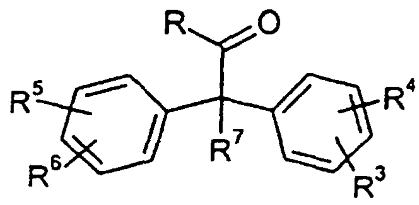

**3**

où les restes $R^3$, $R^4$, $R^5$, $R^6$ et $R^7$ peuvent avoir les significations citées dans les revendications 1 à 4 et R représente le chlore ou un alkyloxy en $C_1$-$C_4$ avec un composé de formule 2

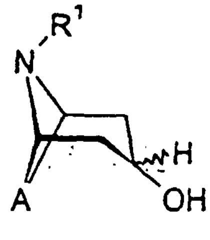

**2**

où A et $R^1$ peuvent avoir les significations citées dans les revendications 1 à 4 en un composé de formule 4

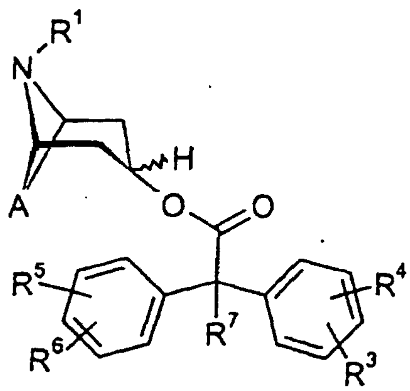

**4**

où A et les restes $R^1$, $R^3$, $R^4$, $R^5$, $R^6$ et $R^7$ peuvent avoir les significations citées dans les revendications 1 à 4, que l'on quaternise ensuite en un composé de formule 1 par réaction avec un composé $R^2$-X où $R^2$ et X peuvent avoir les significations citées dans les revendications 1 à 4.